(19) Europäisches Patentamt / European Patent Office / Office européen des brevets

(11) **EP 3 977 546 B1**

(12) **EUROPEAN PATENT SPECIFICATION**

(45) Date of publication and mention
of the grant of the patent:
**16.10.2024  Bulletin 2024/42**

(21) Application number: **20730701.8**

(22) Date of filing: **29.05.2020**

(51) International Patent Classification (IPC):
*H01M 4/587* (2010.01)        *H01M 10/054* (2010.01)
*H01M 10/0567* (2010.01)      *H01M 10/0568* (2010.01)

(52) Cooperative Patent Classification (CPC):
**H01M 4/587; H01M 10/054; H01M 10/0567;**
**H01M 10/0568;** H01M 2300/0025; Y02E 60/10;
Y02E 60/50

(86) International application number:
**PCT/GB2020/051317**

(87) International publication number:
**WO 2020/240209 (03.12.2020 Gazette 2020/49)**

(54) **NON-AQUEOUS ELECTROLYTE COMPOSITIONS**

NICHT-WÄSSRIGE ELEKTROLYTZUSAMMENSETZUNGEN

COMPOSITIONS D'ÉLECTROLYTE NON-AQUEUSES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **29.05.2019   GB 201907612**

(43) Date of publication of application:
**06.04.2022   Bulletin 2022/14**

(60) Divisional application:
**24194371.1**

(73) Proprietor: **Faradion Ltd.**
**Sheffield, South Yorkshire S1 4DP (GB)**

(72) Inventors:
• **BARKER, Jeremy**
**Sheffield South Yorkshire S1 4DP (GB)**
• **RUDOLA, Ashish**
**Sheffield South Yorkshire S1 4DP (GB)**

(74) Representative: **Bingham, Ian Mark**
**The IP Asset Partnership Limited**
**Prama House**
**267 Banbury Road**
**Oxford OX2 7HT (GB)**

(56) References cited:
EP-A1- 2 120 279        EP-A1- 3 276 735
WO-A1-2018/151674       JP-A- 2014 002 972
JP-A- 2015 069 705      US-A1- 2016 156 069
US-A1- 2016 308 208     US-A1- 2017 309 958
US-A1- 2019 123 390     US-B1- 6 280 883

**Description**

FIELD OF THE INVENTION

The present invention relates to novel non-aqueous electrolyte compositions, a sodium-ion cell comprising said novel non-aqueous electrolyte compositions and energy storage devices such as batteries, rechargeable batteries, electrochemical devices, and electrochromic devices which include said non-aqueous electrolyte compositions.

BACKGROUND OF THE INVENTION

Sodium-ion batteries are analogous in many ways to the lithium-ion batteries that are in common use today; they are both reusable secondary batteries that comprise an anode (negative electrode), a cathode (positive electrode) and an electrolyte material, both are capable of storing energy, and they both charge and discharge via a similar reaction mechanism. When a sodium-ion (or lithium-ion) battery is charging, $Na^+$ (or $Li^+$) ions de-intercalate from the cathode and insert into the anode. Meanwhile charge balancing electrons pass from the cathode through the external circuit containing the charger and into the anode of the battery. During discharge the same process occurs but in the opposite direction.

Lithium-ion battery technology has enjoyed a lot of attention in recent years and provides the preferred portable battery for most electronic devices in use today. However, lithium is not a cheap metal to source and is considered too expensive for use in large scale applications. By contrast sodium-ion battery technology is still in its relative infancy but is seen as advantageous; sodium is much more abundant than lithium and some researchers predict this will provide a cheaper and more durable way to store energy into the future, particularly for large scale applications such as storing energy on the electrical grid. Nevertheless, a lot of work has yet to be done before sodium-ion batteries are a commercial reality.

One area that needs more attention is the development of suitable electrolyte compositions, particularly for sodium-ion batteries.

Although the design of suitable electrolyte compositions is given less attention than active materials (electrodes), their importance should not be overlooked as they are in large part the key to battery life and for determining the practical performance achievable by a cell, for example in terms of capacity, rate capability, safety etc. However, to be a suitable electrolyte composition, it must fulfil a long list of attributes, these include:

- Chemical stability - there must be no reactions during the cell operation, including within the electrolyte itself or with the separator, the electrodes, current collectors or the packaging materials used;
- Electrochemical stability - there must be a wide electrochemical stability window i.e. a large separation between the high and low onset potentials for decomposition by oxidation or reduction;
- Thermal stability - the electrolyte composition must not decompose or chemically break down during normal cell operation and operational temperature
- Physical properties - the electrolyte composition needs to be liquid therefore its melting and boiling points must be well outside the internal operating temperatures of the cell;
- High ionic and low electronic conductivities are necessary to maintain cell operation by $Na^+$ transport and to minimize self-discharge of the cell, respectively;
- Low toxicity;
- Based on sustainable chemistries, i.e. made using abundant elements and via low impact syntheses (energy, pollution etc.) and
- Cost effective production.

In lithium-ion cells, the most common electrolyte compositions contain either $LiPF_6$ or $LiBF_4$ dissolved in organic carbonate-based solvents; electrolyte compositions comprising 1M $LiPF_6$ in either a mixture of EC (ethylene carbonate) / DMC (dimethyl carbonate) or a mixture of EC (ethylene carbonate) / EMC (ethyl methyl carbonate) are regarded by most workers as the "standard" Li-ion cell electrolytes.

In the case of sodium-ion cells, the sodium analogue, $NaPF_6$, may be used in place of the $LiPF_6$, but a much more cost effective alternative is $NaBF_4$; the latter also has the benefit of improved thermal stability compared with $NaPF_6$. Unfortunately, however, $NaBF_4$ has very low solubility in organic carbonate-based electrolyte solvents, and this results in the ionic conductivity of the resulting electrolyte compositions being generally too low for practical application. Thus, poor solubility of $NaBF_4$-containing electrolyte compositions in traditional organic carbonate-based solvents produce inferior electrochemical performance when compared against an equivalent cell using $NaPF_6$.

Furthermore, it should also be stated that although some materials used in lithium systems are able to be carried over to their sodium counterpart, it is by no means safe to assume that this will always be the case, due, in part, to the larger atomic radius of sodium compared to that of lithium. Thus, solvent systems that work for lithium-ion batteries might not work for sodium-ion batteries and vice-versa. A prominent example of this difference is the incompatibility of propylene carbonate (PC) solvent in lithium-ion batteries with graphite anode (the most commonly used anode in commercial lithium-ion systems currently). Due to this incompatibility, commercial lithium-ion batteries typically use EC/DMC or EC/EMC solvent system, as mentioned previously. By contrast, sodium-ion systems will happily tolerate an electrolyte solvent that includes PC. However, it is desirable to find electrolyte solvent systems which are optimal for promoting high sodium-ion battery performance.

The aim of the present invention therefore is to provide improved sodium ion conducting electrolyte compositions (that is, they are electrolyte compositions which are designed for use in sodium-ion secondary cells) which use sodium-containing salts dissolved in a suitable solvent system. The electrolyte compositions of the present invention will be cost effective, will be non-flammable, will demonstrate enhanced separator wettability, particularly with polyolefin separators, and will demonstrate excellent electrochemical performance in sodium-ion cells. The electrolyte compositions of the present invention will be especially useful in sodium-ion cells which employ an anode electrode which comprises a non-graphitic carbon-containing material such as a hard carbon-containing material, or an anode which comprises a sodium insertion material or a conversion-alloying anode material.

The present invention which is defined in the appended claims achieves these aims by providing a novel non-aqueous electrolyte composition comprising:

a) one or more sodium-containing salts selected from the group consisting of sodium tetrafluoroborate ($NaBF_4$) and sodium hexafluorophosphate ($NaPF_6$); and

b) a solvent system which comprises:

i) a first solvent component which consists of one or more organo carbonate-based solvents; and
ii) a second solvent component which consists of one or more surfactants in an amount of >0.5 to ≤10% by weight of the solvent system, wherein the one or more surfactants are selected from the group consisting of: anionic surfactants, cationic surfactants and non-ionic surfactants.

The one or more sodium-containing salts preferably comprise one or more weakly coordinating anions. The term "weakly coordinating anion" or "WCA" is well known to those skilled in the art and is used to refer to anions which comprise several (more than one) elements, which may contain halogen atoms and/or oxygen atoms, and which share a single negative charge. This means that the negative charge is spread out over the anion and makes the coordinating ability of the anion comparatively weak, for example relative to the coordinating ability of an anion which has a concentrated negative charge or one with multiple negative charges. In the disclosure, the sodium-containing salt is selected from one or more compounds of the formula $NaM_mX_x$; one or more compounds of the formula $NaXO_4$; one or more fluoro sulfonyl-containing compounds; one or more fluoro sulfonate-containing compounds; one or more oxalato borate compounds; and compounds that contain a tetrahedral anion such as tetrakis[3,5-bis(trifluoromethyl)phenylborate anion ($B[3,5-(CF_3)_2C_6H_3]^-_4$), tris(pentafluorophenyl)borate anion ($B(C_6F_5)^-_4$), and tetrakis carboxy (trifluoromethyl)aluminate anion ($Al[OC(CF_3)_3]^-_4$).

In the disclosed one or more sodium-containing salts of the formula $NaM_mX_x$, M is one or more metals and/or non-metals and X is a group that comprises or consists of one or more halogens, preferably selected from fluorine, chlorine, bromine and iodine, further preferably fluorine. The amount x of halogen X is preferably x=1 to 16, further preferably x=4 or 6. The amount, m, of the one or more metals and/or non-metals, M, is preferably m=1 to 3, further preferably m=1 to 2, and particularly preferably m=1. Ideally, the one or more metals and/or non-metals, M, is preferably selected from aluminium, boron, gallium, indium, iridium, platinum, scandium, Yttrium, lanthanum, antimony, arsenic and phosphorus. Particularly preferably, M is selected from aluminium, boron, gallium, phosphorus and arsenic. The most preferred sodium-containing salts of the invention are those of the general and selected from sodium tetrafluoroborate ($NaBF_4$), sodium hexafluorophosphate ($NaPF_6$).

In the disclosed one or more sodium compounds of the formula $NaXO_4$, the element X is preferably one or more halogens selected from fluorine, chlorine, bromine and iodine. Chlorine is particularly preferred, and the most preferred sodium-containing salt of the formula $NaXO_4$ is $NaClO_4$.

Disclosed sodium salts of one or more fluoro sulfonyl- and/or sodium salts of one or more fluoro sulfonate-containing compounds include:

- sodium trifluoromethanesulfonate, also known as sodium triflate or NaOTf ($CF_3NaSO_3$);
- Sodium Bis(fluorosulfonyl)imide also known as NaFSI (Na N($SO_2F)_2$); and
- Sodium Bis(trifluoromethanesulfonyl)imide also known as NaTFSI ($C_2F_6NNaO_4S_2$).

Disclosed sodium salts of one or more oxalate borate compounds include:

- sodium bis(oxalate), also known as borate NaBOB or NaB(C2O4)2; and
- Sodium-difluoro(oxalato)borate, also known as NaDFOB.

It is convenient to express the amount of the one or more sodium-containing salts in terms of "the molarity of the components in a) in the solvent system b)"; that is, the total number of moles of the one or more sodium-containing salts, per litre of the solvent system b). Preferably, the molarity of each of the sodium-containing salt individually in a) is in the range 0.1M to 5M, and further preferably in the range 0.1M to $\leq$ 2M. Highly preferably, particularly when the component in a) is $NaBF_4$ or $NaPF_6$, the molarity is in the range 0.1 M to $\leq$ 2M. The total molarity of all of the sodium-containing salts in a) combined, is preferably in the range 0.1 M to 10 M, and more preferably in the range 0.1 M to 6 M, and most preferably in the range 0.1 M to $\leq$ 4 M.

The one or more organo carbonate-based solvents present in the first solvent component i), may be cyclic or non-cyclic compounds that are characterised by the fact that they contain a carbonate ester group, i.e. a carbonyl group that is flanked by one or two alkoxygroups: $R_1O(C=O)OR_2$. The $R_1$ and $R_2$ groups are preferably independently selected (i.e. they may be the same or different from each other) from either hydrogen; or a $C_1$ to $C_{20}$- cyclic or non-cyclic, branched or unbranched, substituted or unsubstituted alkyl group; or a $C_1$ to $C_{20}$- cyclic or non-cyclic, branched or unbranched, substituted or unsubstituted alkenyl group; or a $C_1$ to $C_{20}$- branched or unbranched, substituted or unsubstituted cycloalkyl-, phenyl- or heterocycle-containing group. Highly suitable electrolyte solvents include $C_3$-$C_{10}$ cycloalkyl organo carbonates, such as propylene carbonate ($C_4H_6O_3$) and ethylene carbonate ($C_3H_4O_3$). Propylene carbonate ($C_4H_6O_3$) shows particularly favourable compatibility with electrode materials and the high solubility, wide liquidus range and a high boiling point of this material also makes this solvent advantageous for use in sodium-ion batteries. Other highly suitable organo carbonate-based compounds include diethyl carbonate (DEC), dimethyl carbonate (DMC), ethylene carbonate (EC), ethyl methyl carbonate (EMC), fluoroethylene carbonate (FEC) and vinylene carbonate (VC).

Highly preferred electrolyte compositions of the present invention comprise a first solvent component which consists of propylene carbonate either alone or alternatively in combination with one or more further organo carbonate-based compounds. When a mixture of organo carbonate-based compounds is used, it is convenient to express the amount of each of the one or more organo carbonate-based solvents in terms of a weight ratio. For example, a preferred organo carbonate-based solvent might contain a mixture of ethylene carbonate, diethyl carbonate and propylene carbonate, and this would ideally be in the weight ratio range 1 to 4 : 1 to 10 : 1 to 10 wt/wt and further ideally in the weight ratio 1 to 3 : 1 to 5 : 1 to 5 wt/wt and most ideally 1:2:1 wt/wt.

Extremely advantageous electrolyte compositions of the present invention include propylene carbonate in an amount of at least 40%, preferably at least 50%, further preferably at least 60%, by weight of the organo carbonate-based solvent. Highly preferably propylene carbonate is the sole organo carbonate-based solvent i.e it is present in an amount of 100% by weight of the organo carbonate-based solvent.

An alternative preferred electrolyte composition of the present invention comprises a mixture of ethylene carbonate and diethyl carbonate in the absence of propylene carbonate. These organo carbonate-based components are preferably present in the weight ratio 1 to 20: 1 to 20 wt/wt, further preferably in the weight ratio 1 to 10 : 1 to 10 wt/wt, also preferably 1 to 5 : 1 to 5 wt/wt, and most preferably in the weight ratio 1:1 wt/wt.

The one or more surfactants used in the second solvent component ii) of the present invention are preferably selected to enhance the ability of the electrolyte composition to wet the separator (particularly a polyolefin separator) of the battery which, in turn, advantageously promotes a longer battery cycle life. Such surfactants are ideally one or more selected from:

1) anionic (negatively charged) surfactants. Suitable examples include: carboxylates, such as alkyl carboxylates (e.g. fatty acid salts), carboxylate fluoro-surfactants; sulfates, such as alkyl sulfates (e.g. sodium lauryl sulfate), alkyl ether sulfates (e.g. sodium laureth sulfate); sulfonates: docusates (e.g. dioctyl sodium sulfosuccinate), alkyl benzene sulfonates; phosphate esters such as alkyl aryl ether phosphates and alkyl ether phosphates;

2) Cationic surfactants which bear a positive charge for example $RN^+H_3Cl^-$, $RN^+ (CH_3)_3Cl^-$, diotadecyldimethylammonium chloride, cetyl pyridinium chloride, benzalkonium chloride, hexadecyl trimethylammonium chloride (CTAC) and hexadecyl trimethylammonium bromide (CTAB); and

3) Non-ionic surfactants. These are uncharged, and examples include: polyol esters (e.g. glycol, glycerol esters, sorbitan and sorbitan derivatives such as fatty acid esters of sorbitan (Spans) and their ethoxylated derivatives (Tweens)), polyoxyethylene esters and poloxamers which comprise block copolymers, for example Poloxamer 84, Poloxamer 105, Poloxamer 123, Poloxamer 124, Poloxamer 188, Poloxamer 237, Poloxamer 338, Poloxamer 407 and Poloxamer F127.

Most preferably, the second solvent component ii) of the electrolyte composition of the present invention contains one or more non-ionic block copolymer-containing surfactants.

The total amount of the one or more surfactants used in an electrolyte composition of the present invention, is >0.5 to ≤10% by weight of the solvent system, preferably ≥0.6 to ≤ 6% by weight of the solvent system, further preferably ≥0.6 to ≤4wt%, very preferably ≥0.6 to ≤3wt% and ideally 0.6wt% to ≤2.5wt%, based on the total weight of the electrolyte solvent system used in the electrolyte composition.

In a preferred embodiment, the non-aqueous electrolyte composition according to the present invention further comprises at least one additional compounds which may or may not be a solvent, and is preferably selected from a sulfur-containing compound, a flame retardant compound (such as a polyalkly phosphate-containing compound, preferably a non-fluorinatedpolyalkyl phosphate-containing compound), a diluent (such as a hydrofluoroether-containing compound, preferably a hydrofluoroalkyl ether-containing compound), a glycol diether (also known as a "glyme"), a glycol ether acetate, an ionic liquid, a nitrile-containing compound (such as adiponitrile, glutaronitrile), and any solvent which is capable of promoting the reduction of viscosity of the electrolyte by acting as an inert diluent (such as a hydrofluoroalkyl ether, preferably 1,1,2,2-Tetrafluoroethyl-2,2,3,3-tetrafluoropropyl ether (HFE or TTE)). For the avoidance of doubt, the at least one additional compound does not include any of organo carbonate-based solvents listed in the first solvent component, or a surfactant as listed in the second solvent component.

Suitable sulfur-containing compounds include cyclic and/or non-cyclic sulfur-containing compounds and are preferably selected to enable the formation of a stable cathode-electrolyte interphase (CEI) on the cathode and/or a stable solid-electrolyte interphase (SEI) on the anode, which in turn leads to advantages such as enhanced 1st cycle coulombic efficiencies and cycle life enhancement. Preferably, the one or more sulfur-based compound is a sulfone, i.e. it has a sulfonyl functional group which is attached to two carbon atoms. The central hexavalent sulfur atom is doubly-bonded to each of two oxygen atoms and has a single bond to each of two carbon atoms. The general formula of such compounds is $R\text{-}SO_2\text{-}R'$, in which R and R' may be independently selected (i.e. they may be the same or different from each other) from any straight or branched, substituted or unsubstituted $C_1$ to $C_6$-alkyl group; a straight or branched, substituted or unsubstituted $C_1$ to $C_6$-alkenyl group; a straight or branched, substituted or unsubstituted $C_1$ to $C_6$-alkoxy group; or a substituted or unsubstituted $C_3$-$C_6$-cycloalkyl-, phenyl- or heterocycle-containing group. Further preferably, the sulfur-based compound is selected from a cyclic sulfone, non-cyclic sulfone and a cyclic sultone. Suitable examples include sulfolane $((CH_2)_4SO_2)$, 3-methyl sulfolane $((CH_3)CH(CH_2)_3SO_2),)$ 1.3-propanediolcyclic sulfate (PCS) also known as 1,3,2-Dioxathiane 2,2-dioxide (DTD or $(CH_2)_3SO_4)$), trimethyl sulfone, 1-propene 1,3-sultone $((CH)_2CH_2SO_3)$, 1,3-propane sultone $(CH_2)_3SO_3$, trimethylanesulfone $((CH_2)_3SO_2)$ and methyl phenyl sulfone $((CH_3)(C_6H_5)SO_2)$. One or more cyclic sulfones and/or cyclic sultones and/or 1,3-propanediolcyclic sulfate are particularly preferred. Highly preferably the sulfur-containing compound used in the solvent system does not comprise a sulfoxide-containing compound, a sulfite-containing compound or a sulfide-containing compound, and ideally, the electrolyte composition does not comprise a sulfoxide-containing compound, a sulfite-containing compound or a sulfide-containing compound.

When used in an electrolyte composition of the present invention, the total amount of the one or more sulfur-based compounds is preferably ≤80 wt%, further preferably ≤60 wt%, ideally 0 wt% to ≤60 wt% and most ideally 1 wt% to ≤55 wt%, based on the total weight of the solvent system used in the electrolyte composition.

Suitable optional flame retardant materials include one or more polyalkylphosphate-containing compounds which, when present in the electrolyte composition of the present invention, advantageously not only produce batteries with reduced flammability, but also their use has been discovered to significantly enhance battery cycle life, as well as promoting favourable separator wetting, particularly in the case of polyolefin separator wetting, as compared against conventional organo carbonate electrolyte which lacks a polyalkyl phosphate-containing compound.

Suitable polyalkyl phosphates include trialkyl phosphates and particularly preferably trimethyl phosphate and/or triethyl phosphate.

When used in an electrolyte composition of the present invention, the total amount of the one or more polyalkyl phosphate-containing compounds is preferably at least 1 wt% to 90 wt%, further preferably at least 2 wt% to 70 wt%, more preferably at least 5 wt% to 70 wt% and ideally greater than 10 wt% to 70 wt%, of the total weight of the solvent system used in the electrolyte composition. In some embodiments, an amount of <5wt% of the one or more polyalkyl phosphate-containing compounds is found to be effective to reduce flammability and/or to enhance cycle life and/or to increase wettability of the separator.

Suitable glymes may be selected from: ethylene glycol dimethyl ether (monoglyme CH3-O-CH2 CH2 -O-CH3); diethylene glycol dimethyl ether (diglyme CH3-O-(CH2 -O)2 - CH3); triethylene glycol dimethyl ether (triglyme, CH3-O-(CH2 -O)3-CH3); tetraethylene glycol dimethyl ether (tetraglyme, CH3-O-(CH2 -O)4-CH3); ethylene glycol diethyl ether (ethyl glyme, CH3CH2 -O-CH2 CH2 -O-CH2 CH3); diethylene glycol diethyl ether (ethyl diglyme, CH3CH2 -O-(CH2 -O)2 -CH2 CH3); diethylene glycol dibutyl ether (butyl diglyme, CH3CH2 CH2 -O-(CH2 -O)2 -CH2 CH3); polyethylene glycol) dimethyl ether (polyglyme, CH3-O-(CH2 -O)n-CH3); and dipropylene glycol dimethyl ether (proglyme, CH3-O-(CH2 CHCH3-O)2 -CH3).

Diethylene glycol dimethyl ether (diglyme), triethylene glycol dimethyl ether (triglyme) and tetraethylene glycol dimethyl ether (tetraglyme) are especially preferred.

Suitable one or more glycol ether acetates may be selected from: propylene glycol methyl ether acetate; dipropylene glycol methyl ether acetate; ethylene glycol monobutyl ether acetate; ethylene glycol monomethyl ether acetate; ethylene glycol monoethyl ether acetate; diethylene glycol monobutyl ether acetate; diethylene glycol monoethyl ether acetate; and diethylene glycol monoethyl ether acetate.

Ethylene glycol monomethyl ether acetate and ethylene glycol monoethyl ether acetate are particularly preferred.

Suitable ionic liquids may combine any of the following: cations based on imidazolium, pyrrolidinium, and ammonium families, and anions based on tetrafluoroborate ($BF_4^-$), bis(fluorosulfonyl)imide (FSI), tri(fluoromethanesulfonyl)imide (TFSI), perchlorate ($ClO_4^-$), hexafluorophaphate ($PF_6^-$), and ferric perchlorate ($FeCl4^-$).

The amount of the one or more additional compounds used in the electrolyte compositions of the present invention is preferably in the range 0.1 wt% to 80 wt%, preferably 0.1 wt% to 70 wt%, further preferably 0.2 wt% to 55 wt% and most preferably 0.5 wt% to 50 wt% of the total weight of the solvent system used in the electrolyte composition.

The electrolyte composition according to the present invention may also optionally include one or more performance additives , typically an amount of <15wt%, preferably < 10 wt% and further preferably 0.1 wt% to < 5 wt%, of the total weight of the solvent system used in the electrolyte composition, and preferably act at the electrolyte-electrode interface rather than in the bulk of the electrolyte. Such performance additives are useful, particularly in liquid electrolyte systems where insoluble degradation products form a solid passivation layer on the surface of the negative electrode (SEI), to optimise the ion and electron conductivity of the SEI and also to maximise its strength and adhesion to the surface of the negative electrode. Performance additives are also useful to increase the wetting of the surface of the separator, to protect the cell against overcharging events and to act as fluidity enhancers, viscosity reducers, and/or impurity or radical scavengers. Furthermore, such performance additives may render additional benefits to the cell performance such as providing a fail-safe in case the cell overcharges or impart further non-flammability characteristics to the electrolyte.

Suitable performance additives may be selected from: polymerizable additives for promoting overcharge protection such as biphenyl, diphenylamine, dimethoxydiphenylsilane (DDS), 3-chloroanisole (3CA), N-phenylmaleimide, xylene (methyl-substituted benzene) and cyclohexylbenzene; additives for promoting overcharge protection based on redox-shuttle mechanism such as 2,5-di-tert-butyl-1,4 dimethoxybenzene (DDB), 4-tert-butyl-1,2-dimethoxybenzene (TDB), 1,4 bis(trimethylsilyl)-2,5-dimethoxybenzene (BTMSDB) and 1,4-bis(2-methoxyethoxy)-2,5 di-tert-butylbenzene; additives for imparting further flame retardancy attributes to the electrolyte such as dimethyl methylphosphonate (DMMP), ethoxy-pentafluoro-cyclotriphosphazene ($N_3P_3F_5OCH_2CH_3$, EFPN), tri(2,2,2-trifluoroethyl) phosphite and/or tri(2,2,2-trifluoroethyl) phosphate (TFEP), methyl nonafluorobuyl ether (MFE) and silane-$Al_2O_3$ nanoparticles; and additives for promoting better high temperature cycling such as succinic anhydride.

The electrolyte compositions of the present invention are preferably employed in a sodium-ion cell. Consequently, in a further aspect, the present invention provides a sodium-ion cell comprising a negative electrode, a positive electrode, and an electrolyte composition according to the present invention described above. Such sodium-ion cells may be used in an energy storage device, for example a battery, a rechargeable battery, an electrochemical device and an electrochromic device.

Ideally, the electrolyte compositions of the present invention are used in sodium-ion cells which employ a carbon-containing material as the active negative (anode) electrode material. Preferably the carbon-containing material is a non-graphitisable carbon-containing material. Such carbon materials include, but are not limited to modified graphitic carbon, non-graphitic carbon, isotropic carbon, partially graphitic carbon, exfoliated graphite, expanded graphite, amorphous carbon, non-crystalline carbon, soft carbon and hard carbon materials. For the avoidance of doubt, "non-graphitisable carbon material", as used herein, does not include any carbon with a natural graphite structure in a long-range order (prevalent in the bulk of the structure) or a synthesised graphite material with a structure identical to that of natural graphite material. Typical methods of producing non-graphitisable carbon employ heating starting materials, for example, sucrose, corn starch, glucose, organic polymers (e.g. polyacrylonitrile or resorcinol-formaldehyde gel), cellulose, petroleum coke or pitch coke, to about 1200°C. Commercially available non-graphitisable carbon materials are termed as "hard carbon materials" and include those sold under the name "Carbotron™ hard carbon material" from Kureha Corporation, and "Bio-Carbotron™ hard carbon material" from Kuraray Chemical Company and Kureha Corporation.

Ideally, the sodium-ion cells of the present invention uses anode active materials that contain non-graphitisable carbon (e.g. hard carbon) alone, however it is also advantageous to employ materials that combine the non-graphitisable carbon with one or more other materials (as a mixture or as a composite) such as a Na-storable metal or alloy, or a metal or a non-metal in its elemental or compound form. Particularly preferred anode active materials include hard carbon/X materials, where X may be one or more elements such as antimony, tin, phosphorus, sulfur, boron, aluminium, gallium, indium, germanium, lead, arsenic, bismuth, titanium, molybdenum, selenium, tellurium, silicon or carbon. Hard carbon/Sb, hard carbon/Sn, hard carbon/$Sb_xSn_y$ and hard carbon/P are all suitable hard carbon-containing materials.

The non-graphitisable carbon materials termed as "expanded" or "exfoliated" graphite, have a structure which is recognizable as being similar to the structure of natural graphite but not identical to it because it is modified so that its carbons exhibit an interlayer spacing in the (001) direction which is greater than 3.35 Angstrom.

It is most preferable that the non-aqueous electrolyte compositions of the present invention are used with a non-graphitisable carbon-containing anode, preferably a hard carbon anode.

In a highly preferred embodiment therefore, the present invention provides a non-aqueous electrolyte composition described above which is suitable for use in a sodium-ion cell which contains an anode comprising non-graphitisable carbon material, preferably a hard carbon anode.

In an alternative example, the electrolyte compositions of the present invention may be used in sodium-ion cells which employ an anode which comprises a sodium insertion material such as a titanate compound (either undoped or doped with small quantities of various other metals and/or Ti substituted with other transition metal and/or O substituted with other halogens), for example $Na_2Ti_3O_7$; $NaHTi_3O_7$; $Na_2Ti_6O_{13}$; $Na_2Li_2Ti_5O_{12}$, $K_2Ti_3O_7$, $K_2Ti_6O_{13}$, $TiO_2$, $NaTiO_2$ and $H_2Ti_3O_7$. Other sodium insertion materials such as undoped or doped (with small quantities of various other metals and/or halogens) or partially substituted (with any metal, non-metal, metalloid and/or halogen) $NaTi_2(PO_4)_3$, and $TiS_2$, either in conjunction with, or as an alternative to, a non-graphitisable carbon material.

In a further alternative example, the electrolyte compositions of the present invention may be used in sodium-ion cells which employ an anode which comprises a conversion and/or alloying material and a material that displays conversion and alloying type reaction mechanism to store sodium, for example tin, antimony, molbdenum, phosphorus, sulfur, indium, lead, iron, manganese and germanium, either in elemental form or in compound form, preferably with one or more selected from oxygen, carbon, nitrogen, phosphorus, sulfur, silicon, fluorine, chlorine, bromine and iodine (such as $SnO$, $SnO_2$, $SnF_2$, $SiP_2$, $Fe_2O_3$, $Fe_3O_4$, $FeS$, $FeS_2$, $MoS_2$, $MoOs$, $Sb$, $Sb_2O_3$, $SnSb$ and $SbO$) either in conjunction with, or as an alternative to, a non-graphitisable carbon material.

Secondary carbon-containing materials may also be used in combination with the above mentioned anode active materials, *interalia,* to improve the conductivity of the anode, for example: activated carbon materials, particulate carbon black materials, graphene, carbon nano-tubes and graphite. Example particulate carbon black materials include: "C65™" carbon (also known as Super P™ carbon black) (BET nitrogen surface area 62m$^2$/g) (available from Timcal Limited) although other carbon blacks are also available with a BET nitrogen surface area of <900m$^2$/g, for example "Ensaco 350g™' which is a carbon black with a BET nitrogen surface area of 770m$^2$/g (available from Imerys Graphite and Carbon Limited as speciality carbons for rubber compositions). Carbon nano-tubes have a BET nitrogen surface area of 100-1000m$^2$/g, graphene around 2630m$^2$/g and activated carbon materials have a BET nitrogen surface area of >3000m$^2$/g.

The present invention also provides a sodium-ion cell comprising a negative electrode, a positive electrode and a non-aqueous electrolyte composition according to the present invention as described above, wherein the negative electrode comprises a non-graphitisable carbon material, preferably a hard carbon material. Such sodium-ion cells may be used in an energy storage device, for example a battery, a rechargeable battery, an electrochemical device, and an electrochromic device. The electrolyte compositions according to the present invention find particular utility when employed in a sodium-ion cell which comprises a polyolefin separator.

The sodium-ion secondary cells according to the present invention may comprise any cathode electrode active material, however preferred cathode electrode active materials are of the general formula:

$$A_{1\pm\delta}\, M^1{}_V M^2{}_W M^3{}_X\, M^4{}_Y M^5{}_Z O_{2-c}$$

wherein

A is one or more alkali metals selected from sodium, potassium and lithium;
$M^1$ comprises one or more redox active metals in oxidation state +2, preferably selected from the group consisting of nickel, copper, cobalt and manganese;
$M^2$ comprises a metal in oxidation state greater than 0 to less than or equal to +4;
$M^3$ comprises a metal in oxidation state +2;
$M^4$ comprises a metal in oxidation state greater than 0 to less than or equal to +4;
$M^5$ comprises a metal in oxidation state +3;
wherein

$$0 \leq \delta \leq 1;$$

V is > 0;
W is $\geq$ 0;
X is $\geq$ 0;
Y is $\geq$ 0;

at least one of W and Y is > 0

Z is $\geq$ 0;

C is in the range $0 \leq c < 2$

wherein V, W, X, Y, Z and C are chosen to maintain electrochemical neutrality.

Ideally, metal $M^2$ comprises one or more transition metals, and is preferably selected from manganese, titanium and zirconium; $M^3$ is preferably one or more selected from magnesium, calcium, copper, tin, zinc and cobalt; $M^4$ comprises one or more transition metals, preferably selected from manganese, titanium and zirconium; and $M^5$ is preferably one or more selected from aluminium, iron, cobalt, tin, molybdenum, chromium, vanadium, scandium and yttrium.

A particularly preferred cathode electrode active material will be a nickelate-based material.

An cathode electrode active material with any crystalline structure may be used, however, preferably the structure will be O3 or P2 or a derivative thereof, but, specifically, it is also possible that the cathode electrode active material will comprise a mixture of phases, i.e. it will have a non-uniform structure composed of several different crystalline forms. For example, the cathode active material will comprise a compound with the general formula detailed above in a mixture of O3 and P2 phases. The ratio of O3:P2 phases is preferably 1 to 99: 99 to1.

As demonstrated in the specific examples presented below, the electrolyte compositions of the present invention provide surprising and significant advantages, particularly regarding enhanced cycle life, and enhanced capacity and non-flammability, when compared against electrolyte compositions which fall outside the composition of the present invention, i.e compared against compounds that do not include one or more organo carbonate-based solvents in combination with one or more surfactants as described above.

In an alternative aspect, there is provided a non-aqueous electrolyte composition comprising: a) one or more sodium-containing salts; and b) a solvent system which comprises i) a first solvent component which comprises one or more organo carbonate-based solvents, and a ii) second solvent component which comprises at least one compound selected from, a glyme, a glycol ether acetate, an ionic liquid, a nitrile-containing compound, a diluent (such as a hydrofluoroalkyl ether diluent), a sulfur-containing compound, and a flame retardant (such as a polyalkly phosphate-containing compound); optionally in combination with one or more surfactants. Preferably, suitable glymes, glycol ether acetates, ionic liquids, nitrile-containing compounds, diluents, hydrofluoroalkyl ether diluents, sulfur-containing compounds, flame retardants, polyalkly phosphate-containing compounds and surfactants, are as described above.

In further alternative aspects, there is provided a sodium-ion cell and an energy storage device which comprise a non-aqueous electrolyte composition according to the alternative aspect described above.

BRIEF DESCRIPTION OF THE DRAWINGS

The present invention will now be described with reference to the following figures in which:

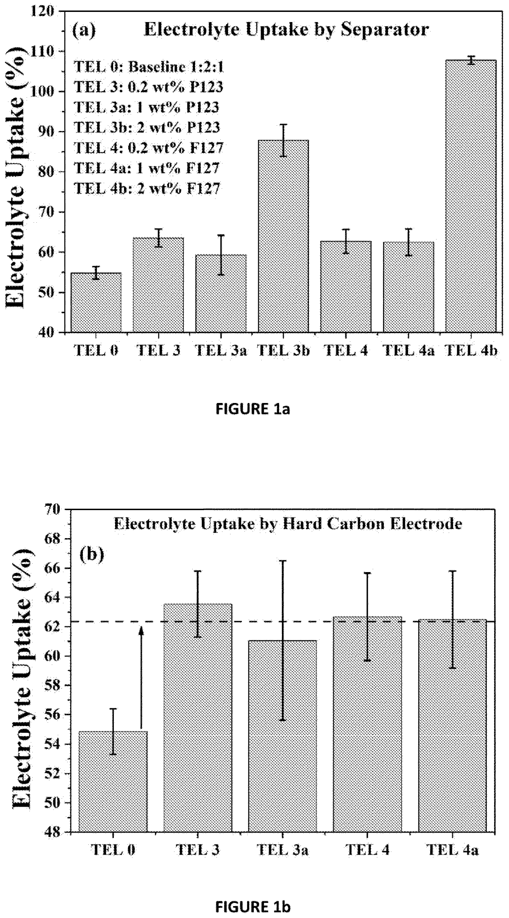

FIGURE 1a shows the uptake by an ND525 separator (Asahi Kasei Corporation) of electrolyte compositions according to the present invention (samples TEL 3, TEL 3a, TEL 3b, TEL 4, TEL 4a and TEL 4b) compared against the uptake of a cell which uses a control electrolyte composition (sample TEL 0).

FIGURE 1b shows electrolyte uptake of electrolyte compositions of the present invention (samples TEL 3, TEL 3a, TEL 4 and TEL 4a), compared with the uptake of a cell which uses a control electrolyte composition (sample TEL 0), by a hard carbon electrode.

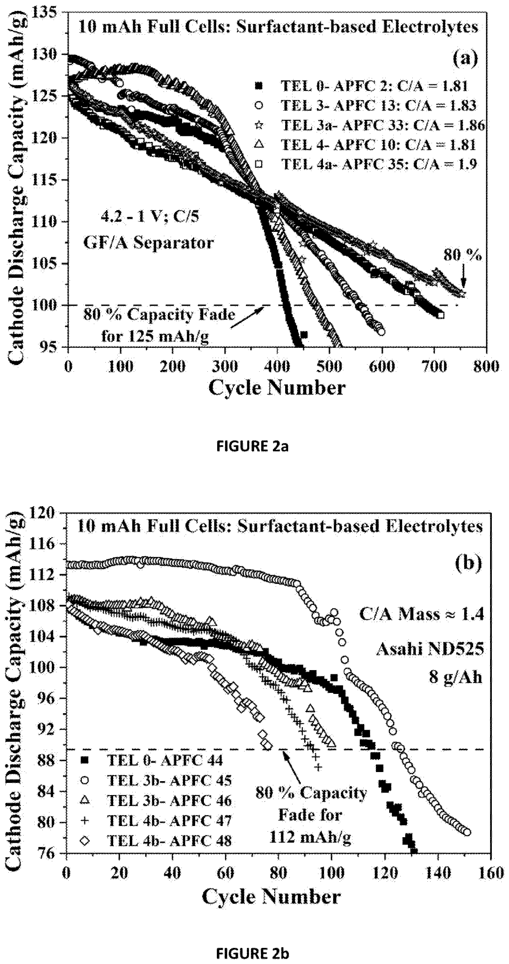

FIGURE 2a shows a plot of cathode discharge capacity (mAh/g) vs cycle number to illustrate the long-term cycling performance at C/5 for 10 mAh cells that use electrolyte compositions of the present invention (samples TEL 3, TEL 3a, TEL 4 and TEL 4a), compared against the cycling performance of a cell which uses a control electrolyte composition (sample TEL 0).

FIGURE 2b shows a plot of cathode discharge capacity (mAh/g) vs cycle number for 10 mAh full cells with a C/A mass balance of 1.4 to provide a comparison of the performance of electrolyte compositions TEL 3b and TEL 4b against the performance of a cell which uses a control electrolyte composition (sample TEL 0).

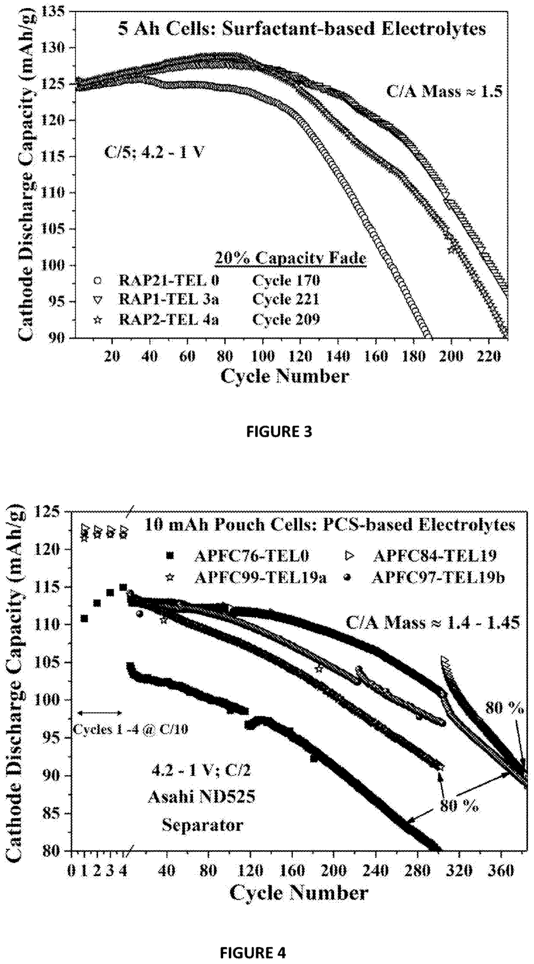

FIGURE 3 shows a plot of cathode discharge capacity (mAh/g) vs cycle number to illustrate the long-term cycling performance of 5 Ah cells that use electrolyte compositions of the present invention (samples TEL 3a, TEL 4a), compared against the cycling performance of a cell which uses a control electrolyte composition (sample TEL 0).

FIGURE 4 shows a plot of cathode discharge capacity (mAh/g) vs cycle number to illustrate the long-term cycling performance of 10 mAh cells that use different electrolyte compositions (samples TEL 19, TEL 19a, TEL 19b all not according to the present invention), compared against the cycling performance of a cell which uses a control electrolyte composition (sample TEL 0).

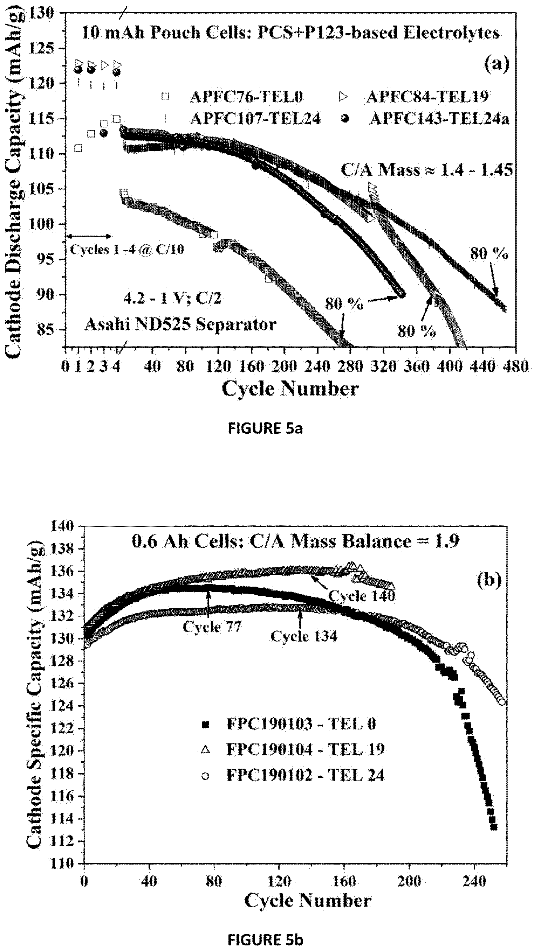

FIGURE 5a shows a plot of cathode discharge capacity (mAh/g) vs cycle number to illustrate the long-term cycling performance of 10 mAh cells containing different electrolyte compositions (samples TEL 19 not according to the present invention and TEL 24, TEL 24a according to the present invention), compared against the cycling performance of a cell which uses a control electrolyte composition (sample TEL 0).

FIGURE 5b shows a plot of cathode specific capacity (mAh/g) vs cycle number to illustrate the long-term cycling performance of 0.6 Ah cells containing different electrolyte compositions (samples TEL 19 not according to the present invention, TEL 24 according to the present invention), compared against the cycling performance of a cell which uses a control electrolyte composition (sample TEL 0).

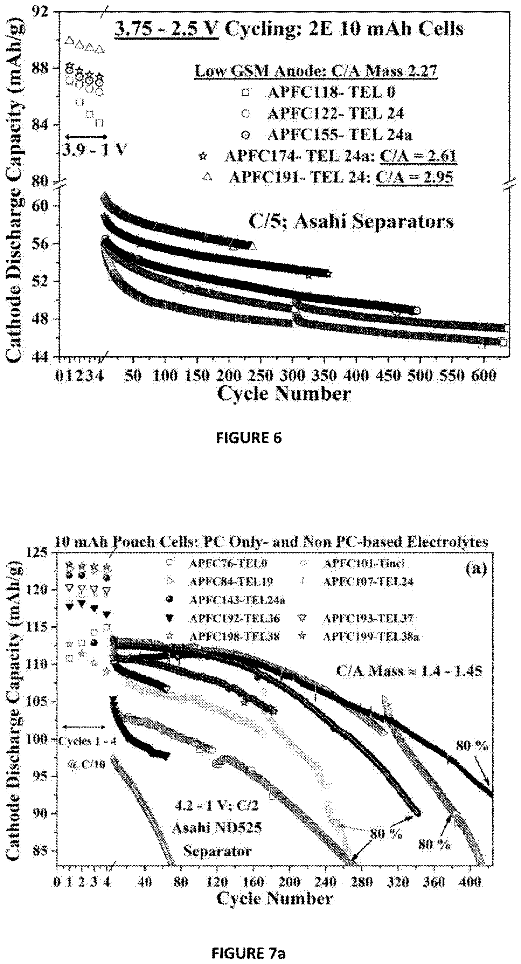

FIGURE 6 shows a plot of cathode discharge capacity (mAh/g) vs cycle number to illustrate the cycling stability within a 3.75 to 2.5 V window for cells that contain different electrolyte compositions of the present invention (samples TEL 24, TEL 24a), compared against the cycling performance of a cell which uses a control electrolyte composition (sample TEL 0).

FIGURE 7a shows a plot of cathode discharge capacity (mAh/g) vs cycle number to illustrate the cycling performance of different electrolyte compositions (samples TEL 19 not according to the present invention and TEL 24, TEL 24a, TEL 37, TEL 38a according to the present invention), within a 4.2 to 1.0 V window, compared against the cycling performance of a cell which uses a control electrolyte compositions (samples TEL 0, TEL 36, TEL 38).

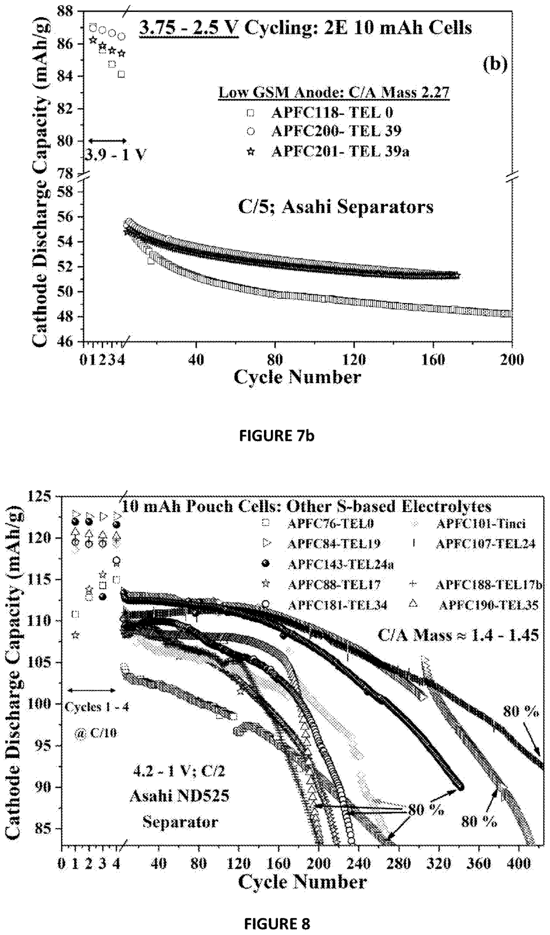

FIGURE 7b shows a plot of cathode discharge capacity (mAh/g) vs cycle number to illustrate the cycling performance of different electrolyte compositions (samples TEL 39 not according to the present invention and 39a according to the present invention), within a 3.75 to 2.5 V window, compared against the cycling performance of a cell which uses a control electrolyte composition (sample TEL 0).

FIGURE 8 shows a plot of cathode discharge capacity (mAh/g) vs cycle number to illustrate the cycling performance of different electrolyte compositions (samples TEL 19, TEL 34 and TEL 35 not according to the present invention and TEL 17, TEL 17b, TEL 24 and TEL 24a, according to the present invention), within a 4.2 to 1.0 V window, compared against the cycling performance of a cell which uses a control electrolyte composition (sample TEL 0).

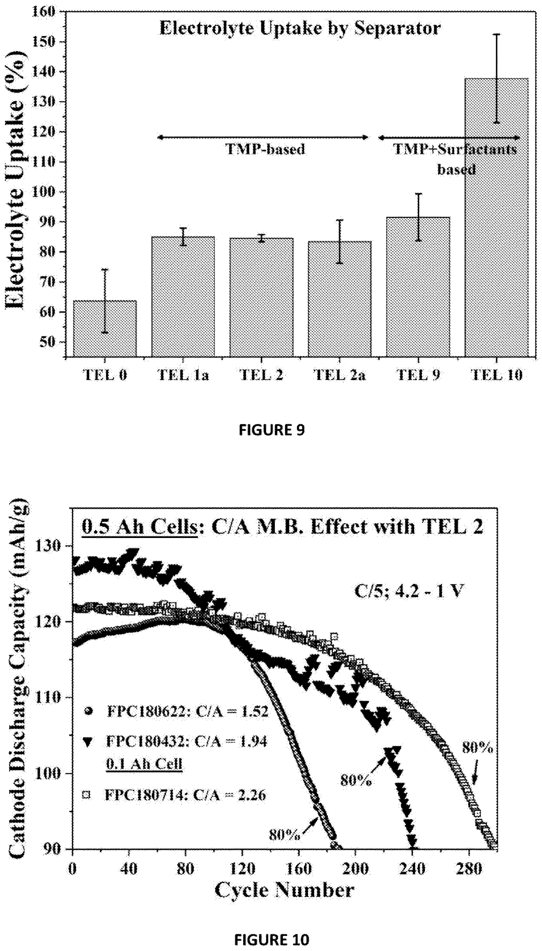

FIGURE 9 shows the electrolyte uptake by an ND525 separator (Asahi Kasei Corporation) of electrolyte compositions (samples TEL 2, TEL 2a, not according to the present invention and TEL 9 and TEL 10 according to thre present invention) compared with the uptake of a cell which uses a control electrolyte compositions (samples TEL 0, TEL 1a).

FIGURE 10 shows a plot of cathode discharge capacity (mAh/g) vs cycle number to illustrate large-scale pouch cell cycling performance of an electrolyte composition (sample TEL 2 not according to the present invention).

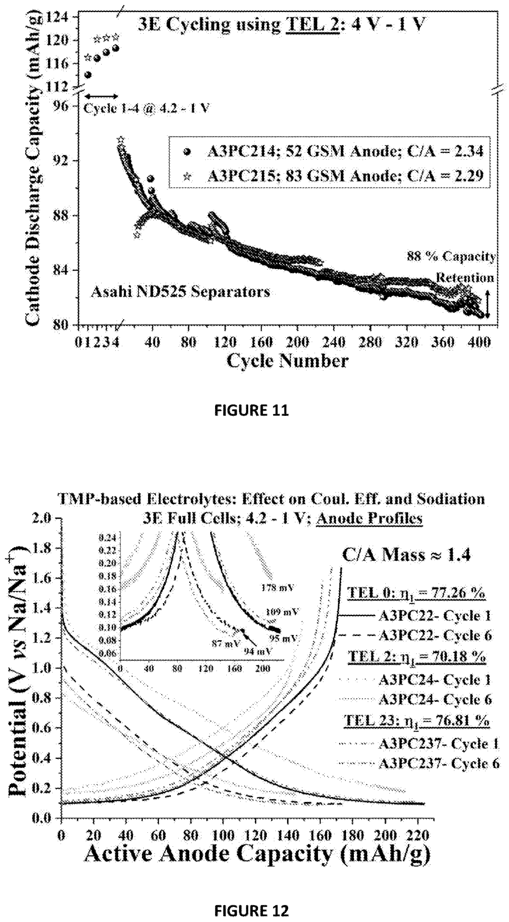

FIGURE 11 shows a plot of cathode discharge capacity (mAh/g) vs cycle number to illustrate the 3E cycling performance of an electrolyte composition (sample TEL 2 not according to the present invention), within a 4.0 to 1.0 V window.

FIGURE 12 shows a plot of potential (V vs Na/Na$^+$) vs active anode capacity (mAh/g) to illustrate the 1$^{st}$ cycle coulombic efficiency performance of electrolyte compositions (samples TEL 2 not according to the present invention, and TEL 23 according to the present invention), in 3E full cells and within a 4.2 to 1.0 V window, compared against the cycling performance of a cell which uses a control electrolyte composition (sample TEL 0).

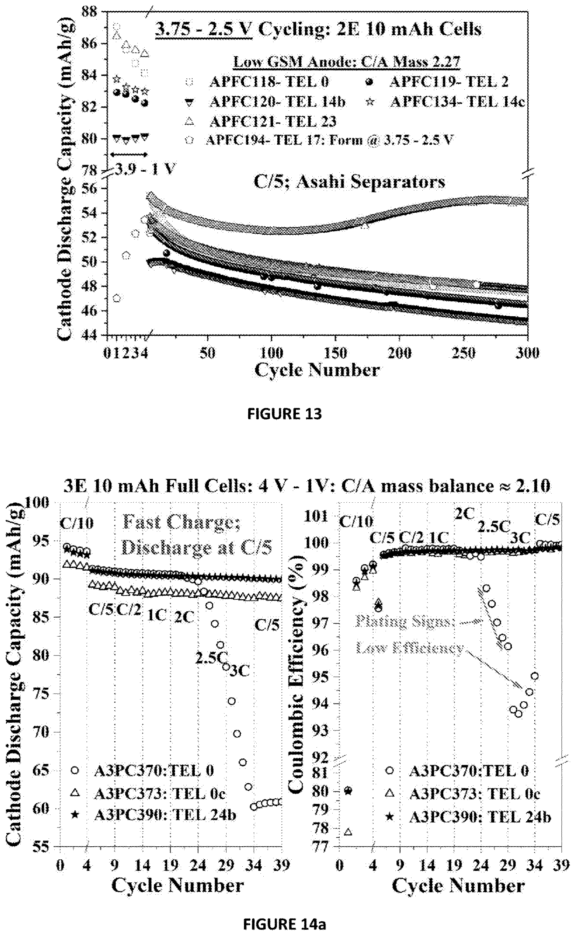

Figure 13 shows a plot of cathode discharge capacity (mAh/g) vs cycle number to illustrate the cycling performance of 10mAh 2E cells which use different electrolyte compositions of the present invention (samples TEL 2 and TEL 14b, not according to the present invention, and TEL 14c, TEL 17 and TEL 23 according to the present invention), compared against the cycling performance of a cell which uses a control electrolyte composition (sample TEL 0).

Figure 14a shows a plot of cathode discharge capacity (mAh/g) vs cycle number and % coulombic efficiency vs cycle number to illustrate the cycling performance of a 10mAh 3E full cell which uses electrolyte composition sample TEL 24b of the present invention, compared against the cycling performance of a cell which uses a control electrolyte compositions (sample TEL 0 and TEL 0c).

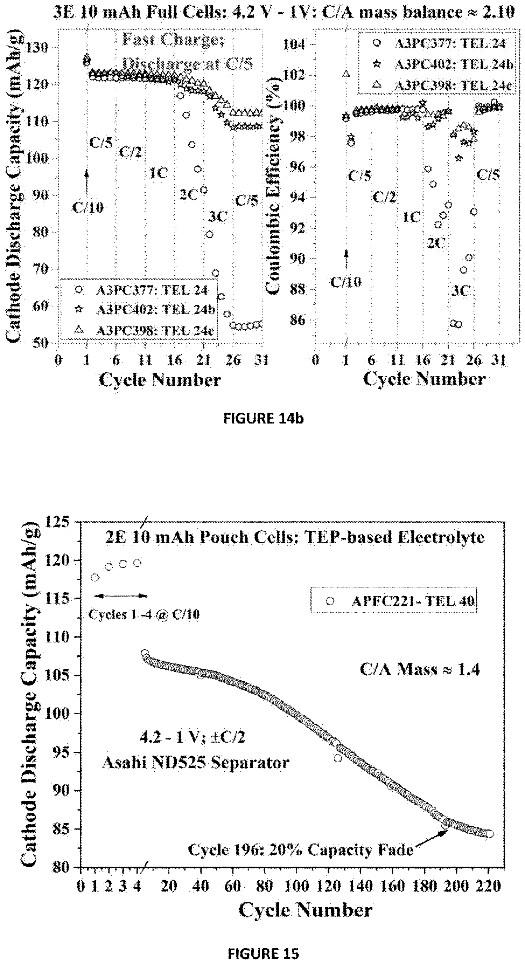

Figure 14b shows a plot of cathode discharge capacity (mAh/g) vs cycle number and % coulombic efficiency vs cycle number to illustrate and compare the cycling performance of 10mAh 3E full cells which use electrolyte compositions, samples TEL 24, TEL24b and TEL 24c of the present invention.

Figure 15 shows a plot of cathode discharge capacity (mAh/g) vs cycle number to illustrate the cycling performance of a 2E 10mAh pouch cell which uses electrolyte composition sample TEL 40 which is not according to the present invention.

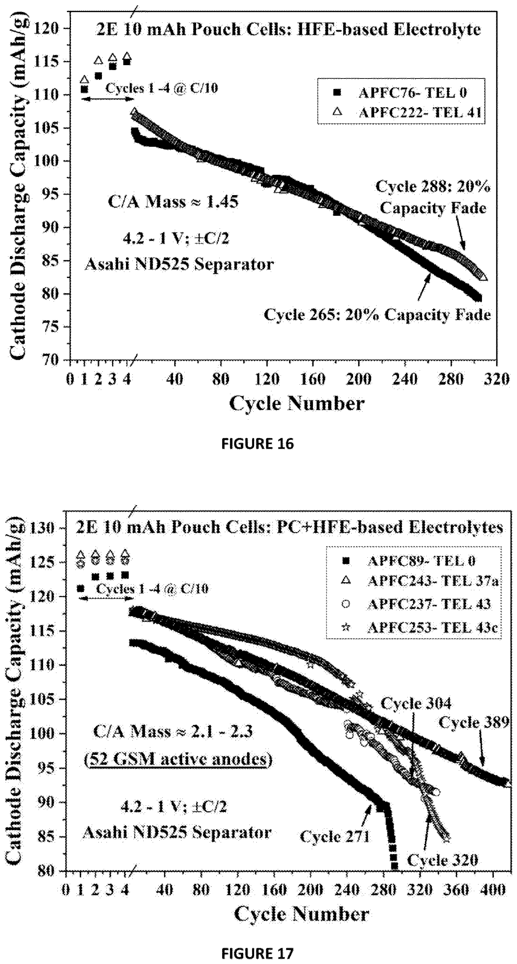

Figure 16 shows a plot of cathode discharge capacity (mAh/g) vs cycle number to illustrate the cycling performance of a 2E 10mAh pouch cell which uses electrolyte composition sample TEL 41 which is not according to the present invention compared against the cycling performance of a cell which uses a control electrolyte composition (sample TEL 0).

Figure 17 shows a plot of cathode discharge capacity (mAh/g) vs cycle number to illustrate the cycling performance of 2E 10mAh pouch cells which use electrolyte compositions, samples TEL 37a, TEL43 and TEL43c of the present invention compared against the cycling performance of a cell which uses a control electrolyte composition (sample TEL 0).

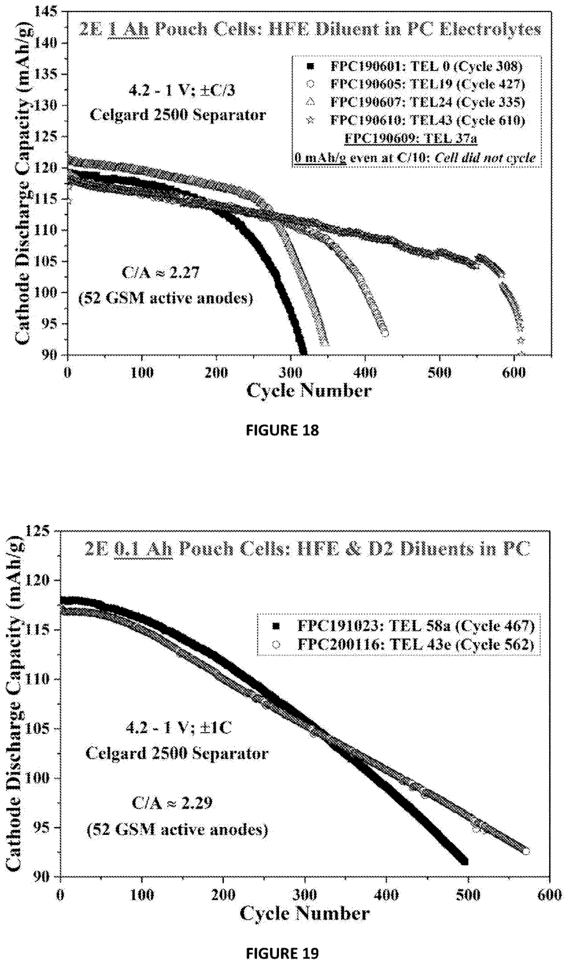

Figure 18 shows a plot of cathode discharge capacity (mAh/g) vs cycle number to illustrate the cycling performance of 2E 1 Ah pouch cells which use electrolyte compositions, samples TEL 19 which is not according to the present invention, and TEL24, TEL 37a and TEL43 which are of the present invention, compared against the cycling performance of a cell which uses a control electrolyte composition (sample TEL 0).

Figure 19 shows a plot of cathode discharge capacity (mAh/g) vs cycle number to illustrate the cycling performance of 2E 0.1 Ah pouch cells which use electrolyte compositions, samples TEL 58a and TEL43e of the present invention.

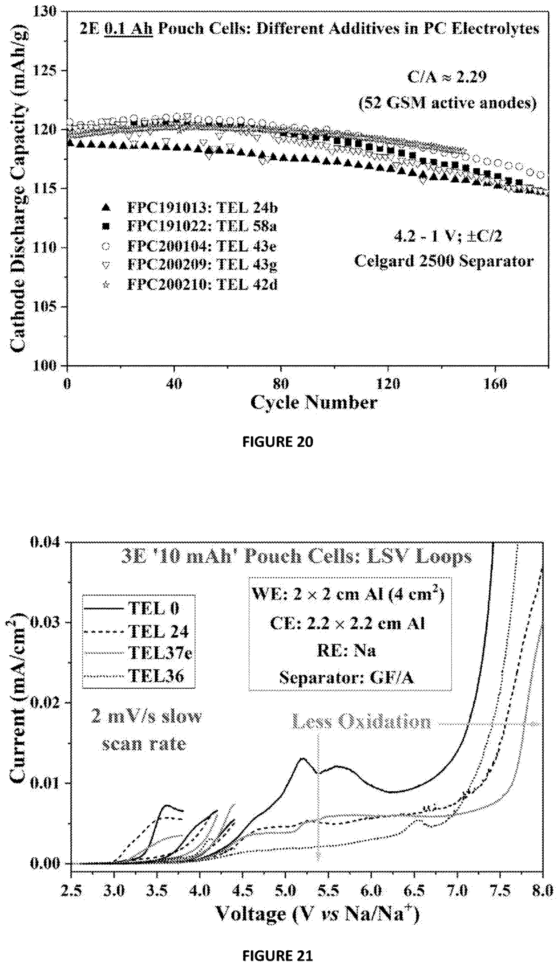

Figure 20 shows a plot of cathode discharge capacity (mAh/g) vs cycle number to illustrate the cycling performance of 2E 0.1 Ah pouch cells which use electrolyte compositions, samples TEL24b, TEL58a, TEL43e, and TEL42d of the present invention and TEL43g which is not according to the present invention.

Figure 21 shows a plot of current (mA/cm$^2$) vs voltage (V vs Na/Na$^+$) to illustrate the cycling performance of 3E 10 mAh pouch cells which use electrolyte compositions, samples TEL24 and TEL37e of the present invention, compared against the cycling performance of a cell which uses control electrolyte compositions (sample TEL 0 and TEL 36).

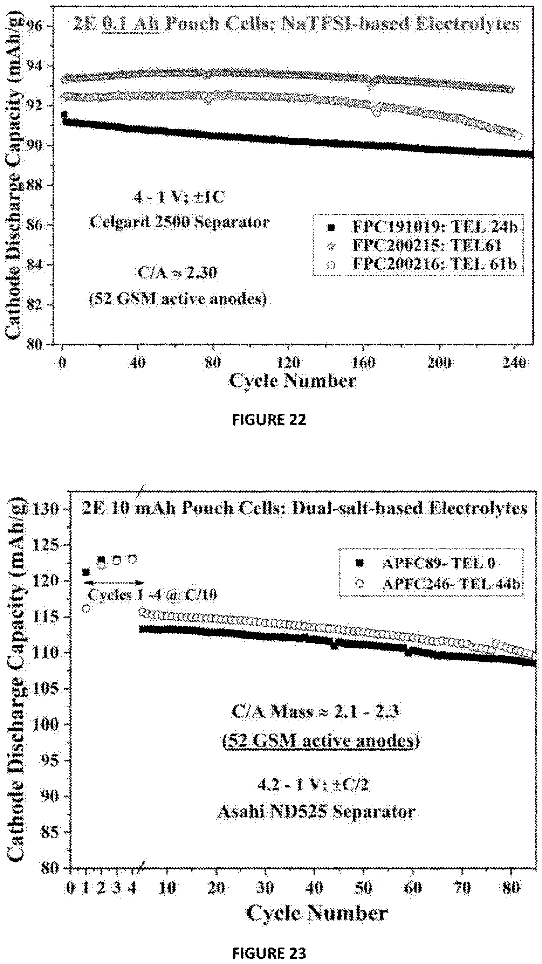

Figure 22 shows a plot of cathode discharge capacity (mAh/g) vs cycle number to illustrate the cycling performance of 2E 0.1 Ah pouch cells which use electrolyte compositions, samples TEL24b, TEL61, and TEL61b of the present invention

Figure 23 shows a plot of cathode discharge capacity (mAh/g) vs cycle number to illustrate the cycling performance of a 2E 10 mAh pouch cell which uses electrolyte composition, sample TEL44b not according to the present invention compared against the cycling performance of a cell which uses a control electrolyte composition (sample TEL 0).

DETAILED DESCRIPTION

The electrolyte compositions under investigation were prepared using the following general procedure: appropriate amounts of the solvents for the desired solvent system were weighed out in an argon-filled glove box and added to a brown glass bottle. To dry the formed solvent thoroughly, 4 Å molecular sieves (Sigma-Aldrich) were added and allowed to dry the solvent mixture for at least 24 h. Afterwards, this solvent mixture was transferred to another brown glass bottle which contained a magnetic stir bar while still in the glove box. The correct amount of one or more sodium-containing compounds was then weighed out in the argon-filled glovebox and slowly added to the solvent mixture under continued stirring. The electrolyte mixture was stirred until the salt was visually determined to have dissolved on a magnetic stirrer plate. The electrolyte mixture was then removed from the stirrer plate and stored and used in the argon-filled glovebox.

The precise composition of each of the electrolyte compositions investigated, is detailed in Table 1 below:

**TABLE 1**

| Electrolyte Composition Sample Ref. | Composition |
|---|---|
| TEL 0 (CONTROL) | 0.5 m $NaPF_6$ in EC:DEC:PC = 1:2:1 wt/wt |
| TEL 0c (CONTROL) | 1 m $NaPF_6$ in EC:DEC:PC = 1:2:1 wt/wt |
| TEL 0+ (CONTROL) | 0.5 m $NaPF_6$ in EC:DEC:PC = 1:3:1 wt/wt |
| TEL 1a (CONTROL) | 0.5 m $NaPF_6$ in TMP |
| TEL 2 (not according to the present invention) | 0.5 m $NaPF_6$ in (EC:DEC:PC = 1:2:1):TMP = 1:1 wt/wt |
| TEL 2a (not according to the present invention) | 0.5 m $NaPF_6$ in (EC:DEC:PC = 1:3:1):TMP = 1:1 wt/wt |
| TEL 3 | TEL 0 with 0.2 wt% P123 |
| TEL 3a | TEL 0 with 1.0 wt% P123 |
| TEL 3b | TEL 0 with 2.0 wt% P123 |
| TEL 4 | TEL 0 with 0.2 wt% F127 |
| TEL 4a | TEL 0 with 1.0 wt% F127 |
| TEL 4b | TEL 0 with 2.0 wt% F127 |
| TEL 9 | TEL 2 with 1.0 wt% P123 |
| TEL 10 | TEL 2 with 1.0 wt% F127 |
| TEL 14b (not according to the present invention) | 0.5 m $NaBF_4$ in (EC:DEC:PC = 1:2:1 wt/wt):TMP = 1:1 wt/wt |
| TEL 14c | TEL 14b with 1 wt% PCS and 1wt% P123 |
| TEL 14d | 1 m $NaBF_4$ in (EC:DEC:PC = 1:2:1 wt/wt):TMP = 1:1 wt/wt with 1 wt% PCS and 1wt% P123 |
| TEL 17 | 0.5 m $NaPF_6$ in (EC:DEC:PC = 1:2:1):Sulfolane = 1:1 wt/wt with 1wt% P123 |
| TEL 17b | TEL 0 with 5 wt% Sulfolane and 1 wt% P123 |
| TEL 19 (not according to the present invention) | TEL 0 with 1 wt% PCS |
| TEL 19a (not according to the present invention) | TEL 0 with 2 wt% PCS |
| TEL 19b (not according to the present invention) | TEL 0 with 3 wt% PCS |
| TEL 23 | TEL 2 with 1 wt% PCS and 1 wt% P123 |
| TEL 24 | TEL 0 with 1 wt% PCS and 1 wt% P123 |
| TEL 24a | TEL 0 with 0.5 wt% PCS and 0.5wt% P123 |
| TEL 24b | TEL 24 with 1 m $NaPF_6$ |
| TEL 24c | TEL 24 with 1.5 m $NaPF_6$ |
| TEL 34 (not according to the present invention) | TEL 0 with 1 wt% 1-Propene 1,3-Sultone |
| TEL 35 (not according to the present invention) | TEL 0 with 1 wt% 1,3-Propanesultone |
| TEL 36 (CONTROL) | 0.5 m $NaPF_6$ in PC |

(continued)

| Electrolyte Composition Sample Ref. | Composition |
|---|---|
| TEL 37 | TEL 36 with 1 wt% PCS and 1 wt% P123 |
| TEL 37a | TEL 36 with 2 wt% PCS and 1 wt% P123 |
| TEL 37e | TEL 36 with 1 wt% PCS, 1 wt% P123 and 5 wt% EC |
| TEL 38 (CONTROL) | 0.5 m NaPF$_6$ in EC:DEC = 1:1 wt/wt |
| TEL 38a | TEL 38 with 1 wt% PCS and 1 wt% P123 |
| TEL 39 (not according to the present invention) | 0.5 m NaPF$_6$ in (EC:DEC = 1:1 wt/wt):TMP = 1:1 wt/wt |
| TEL 39a | TEL 39 with 1 wt% PCS and 1 wt% P123 |
| TEL 40 (not according to the present invention) | 0.5 m NaPF6 in (EC:DEC:PC = 1:2:1 wt/wt):TEP = 1:1 wt/wt |
| TEL 41 (not according to the present invention) | 0.5 m NaPF6 in (EC:DEC:PC = 1:2:1 wt/wt):HFE = 1:1 wt/wt |
| TEL 42d | 1 m NaPF6 in PC with 1 wt% PCS, 1 wt% P123, 1 wt% TMSB and 20 wt% HFE |
| TEL 43 | 0.5 m NaPF6 in PC with 2 wt% PCS, 1 wt% P123 and 20 wt% HFE |
| TEL 43c | 1 m NaPF6 in PC with 2 wt% PCS, 1 wt% P123 and 40 wt% HFE |
| TEL 43e | 1 m NaPF6 in PC with 2 wt% PCS, 1 wt% P123 and 20 wt% HFE |
| TEL 43g (not according to the present invention) | 1 m NaPF6 in PC with 2.5 wt% EC and 20 wt% HFE |
| TEL 44b (not according to the present invention) | 0.5 m NaPF6 + 0.045m NaBF4 in (EC:DEC:PC = 1:2:1 wt/wt):HFE: Tetraglyme = 8:2:1 wt/wt |
| TEL 49d | 1 m NaBF4 in (PC:TMP = 1:1 wt/wt) with 3 wt% PCS, 1 wt% P123 and 20 wt% HFE |
| TEL 56 | 1 m NaPF6 + 0.05 m CsPF6 in EC:DEC:PC = 1:2:1 wt/wt with 1 wt% PCS and 1 wt% P123 |
| TEL 58a | 1 m NaPF6 in PC with 2 wt% PCS, 1 wt% P123 and 20 wt% D2 |
| TEL 61 | 1 m NaTFSI in EC:DEC:PC = 1:2:1 wt/wt with 1 wt% PCS and 1 wt% P123 |
| TEL 61a | 1 m NaTFSI in PC with 2 wt% PCS, 1 wt% P123 and 20 wt% HFE |
| LP 30 (CONTROL) | 1 m LiPF6 in EC:DMC = 1:1 v/v |
| 151 (CONTROL) | 1 m NaPF6 in EC:EMC:PC = 1:5:1 wt/wt |

**Abbreviations used:**

EC = Ethylene carbonate, **DEC** = Diethyl carbonate, **PC** = Propylene carbonate, **TMP** = Trimethyl phosphate, **PCS** = 1,3-propanediolcyclic sulfate, **P123** = Poloxamer (Pluronic) P123, **F127** = Poloxamer (Pluronic) F127, **TEP** = Triethyl phosphate, **HFE** = 1,1,2,2-Tetrafluoroethyl 2,2,3,3-tetrafluoropropyl ether, **TMSB** = Tris(trimethylsilyl) borate, **D2** = 1,1,2,2-Tetrafluoroethyl 2,2,2-trifluoroethyl ether, **Tetraglyme** = Tetraethylene glycol dimethyl ether.

Please note that in the above Table 1, the wt% of the various solvents and/or additives are mentioned with respect to the total solvent weight (they do not account for the weight(s) of the one or more sodium containing salts).

Cell Construction

Generic Procedure to Make a Hard Carbon Na-ion Cell

Sodium ion pouch cells were built using active material electrodes, separator and electrolyte; aluminium tabs were connected to each of the electrodes and the cell was encased in a polymer-coated aluminium pouch.

The positive (cathode) electrode is prepared by solvent-casting a slurry of the active material, conductive carbon, binder and solvent. The conductive carbon used is C65 (Imerys). PVdF co-polymer (e.g. W#7500 from Kureha Chemicals) is used as the binder, and NMP is employed as the solvent. The slurry is then cast onto carbon-coated aluminium foil and dried at about 80°C. The electrode film contains the following components, expressed in percent by weight: 89% active material, 5% C65 carbon, and 6% W#7500 binder.

The hard carbon negative (anode) electrode is prepared by solvent-casting a slurry of the hard carbon active material (Kuranode Type 1, supplied by Kureha), conductive carbon, binder and solvent. The conductive carbon used is C65 (Imerys). PVdF co-polymer (e.g. W#9300, Kureha Chemicals) is used as the binder, and NMP is employed as the solvent. The slurry is then cast onto carbon-coated aluminium foil and dried at about 80°C. The electrode film contains the following components, expressed in percent by weight: 88% active material, 3% C65 carbon, and 9% W#9300 binder.

Cell Testing

The cells are tested as follows using Constant Current (Galvanostatic) Cycling techniques.

The cell is cycled at a given current density between pre-set voltage limits (at top of charge, a constant voltage step is administered). A commercial battery cycler from Maccor Inc. (Tulsa, OK, USA) is used). On charge, alkali ions are inserted into the anode material. During discharge, alkali ions are extracted from the anode and reinserted into the cathode active material.

**Experiment 1: An investigation into the effect of a surfactant on the uptake of the electrolyte composition by a separator and on the wettability by the electrolyte composition on a hard carbon anode electrode.**

Two block copolymer non-ionic surfactant materials were investigated, Poloxamer (Pluronic) P123 and Poloxamer (Pluronic) F127. Electrolyte compositions TEL 3, TEL 3a, TEL 3b, TEL 4, TEL 4a, TEL 4b, TEL 9, TEL 10, as indicated in Table 1 above, were prepared, and the up-take of each of these samples by a separator (Asahi ND525) and by a hard carbon electrode (Kuraray's Type 1 hard carbon) was measured and the results compared against the uptake of a non-surfactant-containing control electrolyte composition, sample TEL 0.

The electrolyte uptake values were calculated using:

$$Electrolyte\ Uptake\ (\%) = \left(\frac{M - M_0}{M_0}\right) \times 100$$

Where, $M_0$ was the weight of the bare separator (or hard carbon electrode) and M was the weight of the separator (or hard carbon electrode) after it was allowed to soak the electrolyte. For this purpose, all separators/electrodes were submerged in a pool of electrolyte for 1 h. The standard deviation is calculated from 3 different measurements:

As the results in Figures 1a and 1b demonstrate, the electrolyte uptake on both the separator and the hard carbon (respectively) increased in the case of the surfactant-containing electrolytes. Specifically, it is apparent from Figures 1a and 1b that adding either 0.2, 1 or 2 wt% of P123 or F127 surfactants to the TEL 0 electrolyte led to electrolyte uptake enhancement of 5 - 50 % on the separator and an enhancement of around 9 % on the hard carbon electrode.

These are statistically significant values and Example 2 below was used to test whether or not improved up-take translates into better electrochemical performance.

**Experiment 2: The cycling performance of cells which use an electrolyte composition that contains a surfactant.**

Using the generic method described above, each of the sample electrolyte compositions TEL 3, TEL 3a, TEL 4, TEL 4a, and the control electrolyte composition TEL 0, were employed to prepare 10 mAh test cells. The electrochemical performance of each test cell was then investigated by cycling between 4.2 and 1.0 V. From the results shown in Figure 2a, it is apparent that 1 wt% of either P123 or F127 delivered a longer cycle life than 0.2 wt % P123 or F127 and that both of these wt% amounts of surfactant outperformed the performance of TEL 0. The best performance was observed for the cell which contained the electrolyte composition TEL 3a (1 wt% P123) and this was marginally better than the performance of TEL 4a (1wt% F127).

From Figure 2b, it can be seen that further increasing the surfactant additive to 2 wt% significantly reduced performance and that samples TEL 3b and TEL 4b resulted in a similar worsening of performance compared against the results for a cell that used control electrolyte composition TEL 0.

Figure 3 shows the long-term cycling data on large-scale 5 Ah cells using either TEL 0, TEL 3a or TEL 4a electrolyte compositions, and confirms the trend of cycle life enhancement when a 1 wt% P123 or F127 surfactant is used, even in a large-scale 5 Ah cell. A cycle life enhancement of 51 cycles was also observed for the cell which used electrolyte composition TEL 3a as compared against a similar cell which used electrolyte composition TEL 0.

Taken in combination, the results shown in Figures 2 and 3, lead to the conclusion that 1 wt % P123 surfactant additive (TEL 3a) give substantial improvements in electrochemical performance.

**Experiment 3: Investigation of the cycling performance of cells which use an electrolyte composition that contains a sulfur-containing compound.**

1,3-Propanediolcyclic sulfate (PCS) was added to the control electrolyte composition TEL 0 to produce electrolyte composition samples, namely: TEL 19 (1 wt% PCS), TEL 19a (2 wt% PCS) and TEL 19b (3 wt% PCS), not according to the present invention

As shown in Figure 4, all of the PCS-containing samples (TEL 19, TEL 19a and TEL 19b) gave better cycling performance compared against the performance by the control electrolyte composition sample TEL 0, both in terms of delivered capacity (a capacity enhancement of around 10 mAh/g higher than that observed for the cell which used TEL 0) and cycle life (a cycle life enhancement of 40 - 120 cycles was obtained compared with the cell which used TEL 0).

It was also observed that PCS-containing samples gave a $1^{st}$ cycle coulombic efficiency which increased from 77 % (for the cell which used the TEL 0 electrolyte composition) to 80 % (for cells which used the TEL 19, 19a and 19b electrolyte compositions). This increase in performance could explain the higher capacities observed for PCS-based electrolytes as lesser amount of $Na^+$ from the cathode was irreversibly consumed.

The best cycling performance was observed for the cell which used an electrolyte composition which contained 1 wt % PCS additive (sample TEL 19).

**Experiment 4a: Investigation of the cycling performance of 10 mAh cells which use an electrolyte composition that contain a sulfur-containing compound (PCS) and a surfactant.**

The long-term cycling performance was investigated for 10 mAh cells which contained either the electrolyte composition of TEL 0 (control), or the composition of TEL 0 with 1% PCS (sample TEL 19), or the composition of TEL 0 and 1wt% PCS + 1wt% P123 (sample TEL 24), or the composition of TEL 0 and 0.5 wt% PCS + 0.5 wt% P123 (sample TEL 24a).

From Figure 5a, it can be seen that the addition of a sulfur-containing compound, PCS, and a surfactant, Poloxamer P123, led to improved cycling performance compared against the cycling performance of cells containing the control electrolyte composition TEL 0. It is particularly striking that the cell containing the TEL 24 electrolyte composition cell even outperformed the cell which contained the TEL 19 electrolyte composition by 72 cycles. Moreover, the cell containing the TEL 24 cell electrolyte composition resulted in a cycle life enhancement of 191 cycles (a 72 % enhancement) over the TEL 0 control cell. But it can be seen that TEL 24 was the best performing sample highlighting the benefit of increasing the surfactant wt% from 0.5 wt% (for TEL 24a) to 1 wt% (for TEL 24).

**Experiment 4b: Investigation of the cycling performance of 0.6 Ah cells which use electrolyte compositions that contain a sulfur-containing compound (PCS) and a surfactant.**

The long-term cycling performance was further investigated for larger-scale 0.6 Ah cells which contained either the electrolyte composition of TEL 0 (control), or the compositions of the present invention: TEL 0 with 1% PCS (sample TEL 19), or the composition of TEL 0 and 1wt% PCS + 1wt% P123 (sample TEL 24).

As shown in Figure 5b, the good performance of TEL 24, shown above in Experiment 5a, was also achieved with larger 0.6 Ah cells. Further, from the results presented in Figure 5b it is believed that the cycle lives of cells using 1 wt% PCS additive (either TEL 19 or TEL 24) is likely to be longer than that of the cell using TEL 0, this is based on the position of a maxima in the capacity vs cycle number indicated as a 'hump' in the plots in Figure 5b. Specifically, it is noted that the position of the 'hump' along the x-axis increased from cycle 77 for TEL 0, to cycle 134 for TEL 24 (an increase of 57 cycles). In respect of the TEL 19 cell, maximal point was reached after 140 cycles.

Figure 6 shows the cycling stability within a 3.75 - 2.5 V window to simulate the voltage profile for Starter-Ignition-Lighting (SLI) batteries currently used in automobiles. In particular, for cells with a C/A mass balance of 2.27, the electrolyte compositions TEL 24 and TEL 24a led to significantly higher capacities within a 3.75 - 2.5 V window, than a similar control cell which used electrolyte composition TEL 0 cycled under similar voltage conditions. Specifically, the delivered capacity at cycle 60 for the control cell using electrolyte composition TEL 0 is 50.4 mAh/g, whereas the cell using electrolyte composition TEL 24 delivered 53.1 mAh/g and the cell using electrolyte composition TEL 24a delivered 54.3 mAh/g.

Advantageously, it was also found that the delivered capacity could be further enhanced by increasing the C/A mass balance from 2.27 to 2.61 and 2.95.

**Experiment 5a: Investigation into the effect on the cycling performance of 10 mAh cells by the inclusion of propylene carbonate as the only organo carbonate-based solvent in the electrolyte composition.**

The long-term cycling performance was investigated, between 4.2 to 1 V for 10 mAh, for cells which used an electrolyte composition that contained either pure propylene carbonate (control sample TEL 36), or a mixture of propylene carbonate and 1 wt% PCS and 1wt% P123, (sample TEL 37), or the control electrolyte composition (control sample TEL 0).

As the results in Figure 7a show, an electrolyte composition which contained propylene carbonate as the only organo carbonate-based solvent (and also no other second solvent component additive), control sample TEL 36, delivered inferior cycling stability as compared against a cell that used the control electrolyte TEL 0. However, an electrolyte composition that contained propylene carbonate as the only organo carbonate-based solvent but which also contained 1 wt % PCS and 1 wt % P123 additives (sample TEL 37) delivered higher initial discharge capacities and also better cycling stability over 60 cycles, as compared against a similar cell using the control electrolyte TEL 0. This result demonstrates that pure PC-based electrolyte compositions are particularly effective in hard carbon-based sodium-ion cells, when a surfactant and a sulfur-containing additive is also present.

**Experiment 5b: Investigation into the effect on the cycling performance of 10 mAh cells by the use of an electrolyte composition from which propylene carbonate is absent.**

The long-term cycling performance between 4.2 to 1 V was investigated for 10 mAh cells which used an electrolyte composition with a first solvent component containing either ethylene carbonate and diethyl carbonate (i.e. no propylene carbonate) (control sample TEL 38), or ethylene carbonate and diethyl carbonate (i.e. no propylene carbonate) with 1 wt% PCS and 1 wt% P123, (sample TEL 38a), or the control electrolyte composition (sample TEL 0).

As shown in Figure 7a, the cycling performance of a non-PC based electrolyte composition including 1 wt% PCS and 1 wt% Ploxamer P123 (sample TEL 38a) delivered a markedly higher capacity (112.4 mAh/g) in the 5th cycle (after 4 formation cycles at C/10) and significantly enhanced cycling stability in respect of a comparable cell that used an electrolyte composition with identical EC:DEC solvents but without the PCS and surfactant addition (sample TEL 38) which showed much lower 5th cycle capacity (97.5 mAh/g) with very poor cycling stability. This result demonstrates that the electrochemical performance of organic carbonate-containing electrolyte compositions can be significantly enhanced by the inclusion of a sulfur-containing compound and a surfactant additive.

The long-term cycling performance between 3.75-2.5 V was also investigated for two electrode (2E) 10 mAh cells which used an electrolyte composition with a first solvent component containing either ethylene carbonate and diethyl carbonate (i.e. no propylene carbonate) and TMP (1 : 1 wt/wt) (sample TEL 39), not according to the present invention, or ethylene carbonate and diethyl carbonate (i.e. no propylene carbonate) and TMP (1 : 1 wt/wt) (sample TEL 39) with 1 wt% PCS and 1 wt% P123, (sample TEL 39a according to the present invention), or the control electrolyte composition (sample TEL 0).

As the results in Figure 7b show, non PC-based TELs, TEL 39 and TEL 39a (using 50 wt% of EC:DEC solvent and 50 wt% TMP) exhibited stable and noticeably higher capacities in the initial cycle with respect to the TEL 0 cell, thereby indicating that TMP-based electrolytes are highly compatible with non PC-based solvents. The results also confirm that the inclusion of PCS and P123 additives resulted in similarly stable cycling and higher capacities, and further demonstrate that such non PC-based electrolyte compositions are likely to be useful for Starter-Ignition-Lighting (SLI) batteries.

**Experiment 6: Investigation into the effect on the cycling performance of 10 mAh cells of using different sulfur-containing compounds in their electrolyte composition.**

The long-term cycling performance was investigated between 4.2 to 1 V for 10 mAh cells which used an electrolyte composition which contained either a 1:1 wt/wt ratio of organo carbonate : sulfolane with 1 wt% of the surfactant P123 (sample TEL 17), or a mixture of 5 wt% sulfolane and 1 wt% surfactant P123 (sample TEL 17b), or 1 wt% 1,3-propanediolcyclic sulfate (sample TEL 19, not according to the present invention) or 1 wt% 1-propene 1,3-sultone (sample 34), or 1 wt% 1,3 propanesultone (sample TEL 35, not according to the present invention), or the control electrolyte composition (control sample TEL 0).

As the results in Figure 8 show, electrolyte compositions that contain sulfolane-based compounds (samples TEL 17 and TEL 17b) cycle well and, by the presence of sulfolane, are expected to be non-flammable. Although the cycling stability is reduced as compared against the control electrolyte composition TEL 0, this is expected to improve with optimisation of the sulfolane-containing cells. Furthermore, as discussed later in Experiment 11, the cycle life of cells using TEL 17 within the 3.75 - 2.5 V window can be as good as that seen for the control TEL 0 cells (refer to Figure 13), hence, sulfolane-based electrolytes can still show excellent cycling stability. Electrolyte samples 34 and 35 both show an enhanced initial discharge capacity and cycling stability (over 180 cycles) compared against the cycling results for a similar cell that used control electrolyte composition TEL 0.

**Experiment 7: Investigation to test the flammability of trimethyl phosphate-containing electrolyte compositions.**

Four different electrolyte compositions were prepared, one contained the control electrolyte composition TEL 0, another contained the control electrolyte composition TEL 0+, another contained a 1 : 1 wt/wt mixture of (EC:DEC:PC = 1:2:1 wt/wt) : trimethyl phosphate (sample TEL 2), and the fourth contained a 1 : 1 wt/wt mixture of (EC:DEC:PC = 1:3:1 wt/wt) : trimethyl phosphate (sample TEL2a). The same amount of each electrolyte composition was then directly lit on fire with a lighter and the time which elapsed between the moment the electrolyte composition caught fire and the moment it completely self-extinguished was recorded and used to calculate the "Self-Extinguishing Time" (SET). Each of the four electrolyte compositions were tested three times and the results used to produce an average SET for each composition. A SET on < 6 s/g correlates to a non-flammable electrolyte composition. The SETs for each of the electrolyte compositions tested are detailed in Table 2 below:

TABLE 2

| Electrolyte | Composition | Wt of 400 μL (9) | Test no. | SET (s) | SET (s/g) | Average SET (s/g) | Standard Deviation (s/g) |
|---|---|---|---|---|---|---|---|
| **TEL 0 (CONTROL)** | 0.5 m NaPF$_6$ in **1:2:1** (EC:DEC:PC) | 0.47 | 1 | 35.75 | 75.76 | 80.93 | 4.76 |
| | | | 2 | 40.17 | 85.13 | | |
| | | | 3 | 38.64 | 81.89 | | |
| **TEL 0+ (CONTROL)** | 0.5 m NaPF$_6$ in **1:3:1** (EC:DEC:PC) | 0.45 | 1 | 50.12 | 110.32 | 126.77 | 14.4 |
| | | | 2 | 62.29 | 137.12 | | |
| | | | 3 | 60.36 | 132.86 | | |
| **TEL 2** (not according to the present invention) | (EC:DEC:PC = 1:2:1 wt/wt) :TMP = 1:1 wt/wt | 0.49 | 1 | 0.1 | 0.21 | 0.18 | 0.039 |
| | | | 2 | 0.1 | 0.2 | | |
| | | | 3 | 0.07 | 0.14 | | |
| **TEL 2a** (not according to the present invention) | (EC:DEC:PC = 1:3:1 wt/wt) :TMP = 1:1 wt/wt | 0.48 | 1 | 0.3 | 0.63 | 0.72 | 0.08 |

It is readily apparent from the results shown in Table 2 that the trimethyl phosphate (TMP)-containing electrolyte compositions samples TEL 2 and TEL 2a, are non-flammable and are therefore highly advantageous over non-TMP-containing compositions.

**Experiment 8: Investigation to test the effect of adding trimethyl phosphate to the electrolyte compositions on up-take by an Ashai ND525 separator.**

Samples of electrolyte compositions, TEL 0, TEL 1a, TEL 2, TEL 2a, TEL 9 and TEL 10 (the compositions as detailed in Table 1 above) were prepared and their up-take by an Ashai ND525 separator was measured.

As shown in Figure 9 all of the TMP-containing electrolyte compositions demonstrated a higher uptake by the separator than the control composition TEL 0. The uptake results for TEL 2 and TEL 2a were about 20% higher than those for TEL 0, but an even greater uptake was observed for the trimethyl phosphate and surfactant-containing composition, samples TEL 9 and TEL 10 with around a 28% and a more than 70% improvement over TEL 0, respectively. Based on these results, it is confidently expected that other TMP + surfactant-based electrolyte compositions, for example TEL 14c and TEL 23, would also demonstrate advantageously high uptake by a separator.

**Experiment 9: Investigation into the electrochemical performance of 0.5 Ah cells that use trimethyl phosphate-containing electrolyte compositions.**

The long-term cycling performance (between 4.2 - 1 V) for 0.5 Ah cells with C/A mass balances 1.52, 1.94 and 2.26 and the electrolyte composition TEL 2 (a combination of electrolyte composition TEL 0 with trimethyl phosphate (TMP) in a solvent ratio of 1:1 wt/wt).
As shown in Figure 10, the cycle life of TMP-containing cells, which use the electrolyte composition TEL 2, is increased from around 185 cycles to around 300 cycles when the mass balance of the cells is increased from 1.52 to 2.26.
As seen from Figure 11, the cycle life can be further enhanced for TMP-based TEL 2 if cycled between 4 V - 1 V. Under these de-rated conditions, both regular GSM Type 1 hard carbon anode (83 GSM active) and low GSM Type 1 hard carbon anode (52 GSM active) demonstrated excellent cycling stability over 400 cycles with capacity retention of 88 %. Hence, within the 4 - 1 V window, TMP-based electrolytes can produce very stable cycling with the added benefit that these sodium-ion cells would also be non-flammable.

**Experiment 10: Investigating the electrochemical performance of electrolyte compositions which contain 1,3-propanediolcyclic sulfate as the sulfur-containing compound and $NaPF_6$ as the sodium-containing compound.**

The 1st cycle coulombic efficiency was investigated between 4.2 to 1 V for three-electrode (3E) full cells which used an electrolyte compositions TEL 2 and TEL 23 and the results were compared against those for a similar cell which used the control TEL 0 electrolyte composition.
As Figure 12 shows, a PCS + P123 + TMP-based electrolyte composition (TEL 23, according to the present invention) produces an improved 1st cycle efficiency as compared with TEL 2 (not according to the present invention) and TEL 0 (control sample).

**Experiment 11: Investigating the electrochemical performance of 2E 10 mAh cells comparing the effects on the electrochemical performance of electrolyte compositions according to the present invention.**

2E 10 mAh cells were prepared using electrolyte compositions TEL 2, TEL 14b, TEL 14c, TEL 17 and TEL 23, and results for the long-term cycling performance (between 3.75 - 2.5 V) were obtained and compared against cycling results of a similar cell using the control electrolyte composition TEL 0.
As mentioned above, a 3.75 - 2.5 V window simulates the voltage profile for Starter-Ignition-Lighting (SLI) batteries currently used in automobiles, therefore is highly advantageous that the results shown in Figure 13 indicate that electrolyte compositions that contain TMP and $NaBF_4$ (samples TEL 14b, TEL 14c) and the composition which contains sulfolane and the surfactant P123 (TEL 17) are very well suited to this end-use application. The electrolyte composition TEL 2 also shows excellent cycling stability over 300 cycles under these voltage conditions.
Thus, it is confirmed that $NaBF_4$ may be used in place of $NaPF_6$ (sample TEL 14b), and that $NaBF_4$-based TMP electrolytes are also compatible with 1wt% PCS and 1 wt% P123 additives (TEL 14c). Significant cost savings and greater flexibility in the choice of electrolyte salts are expected to be provided by the electrolyte compositions of the present invention.

**Experiment 12: Investigating the charge acceptance of sodium-ion cells using electrolyte compositions according to the present invention.**

Charge acceptance is commercially significant for any battery technology as it dictates how quickly a battery can be safely charged. Fast charging increases likelihood of alkali metal plating on the anode in alkali-ion cells (especially if low voltage anodes are used) and this can lead to explosions - a fact that is well known to anyone skilled in the art. Whilst it is generally known in the lithium-ion battery prior art that increasing electrolyte conductivity by increasing salt concentration can influence charge acceptance, such demonstrations have not been shown before in sodium-ion systems. Figure 14a illustrates an example of how the charge acceptance of sodium-ion cells can be enhanced by increasing salt concentrations. In this example, the 3E 10 mAh cells were charged at various rates as indicated on the figure with the discharge cycle always at C/5 (operating temperature was 30 °C). Within the 4 - 1 V window, it can be seen that TEL 0c can enable fast charging at 2.5C or 3C but TEL 0 caused rapid capacity fade beyond 2C due to sodium plating on the hard carbon anode (the low coulombic efficiencies for TEL 0 at 2.5C and 3C shown in the right panel can be taken as a proxy for sodium plating). Furthermore, TEL 24b, an electrolyte composition according to the present invention, achieves even better capacity retention at the fast 2.5C and 3C charge rates than TEL 0c - TEL 24b cell could deliver 90.2 mAh/g if charged at 3C as opposed to 87.5 mAh/g for the TEL 0c cell in like-for-like conditions.
Figure 14b extends the above insights into further embodiments of the present invention to show 3E 10 mAh full cell fast charge cycling data on sodium-ion cells cycled at 4.2 - 1 V using three electrolytes: TEL 24 (0.5 m $NaPF_6$),

TEL 24b (1 m NaPF$_6$), and TEL 24c (1.5 m NaPF$_6$). As before, the three cells were charged at various rates as indicated on the figure with the discharge cycle always at C/5 (operating temperature was 30 °C). While the TEL 24 cell (A3PC377) exhibited a precipitous decline in capacity after 1C, this did not occur for the TEL 24b cell (A3PC402) and the TEL 24c cell (A3PC398). In fact, it can be seen that TEL 24c exhibited the most superior charge acceptance characteristics at varying rates: the discharge capacity of cycle 26 was 112.3 mAh/g for A3PC398 at 3C rate while the corresponding value for A3PC377 was just 54.8 mAh/g, or just 49% of the value obtained for A3PC398.

These results demonstrate that embodiments of the present invention can lead to superior charge acceptance of sodium-ion cells even at fast rates and at varying voltage windows. From these experiments, it should be obvious to anyone skilled in the art that by varying the C/A mass balance and voltage window of the cell, even faster charge acceptance can be achievable with such electrolytes. Furthermore, it will be obvious to anyone skilled in the art that alternate modes of charging such as tapered charging (ultra-fast charge rates such as 4C or 10C to a set %SOC such as 60 or 80%SOC before reducing the charge rate to lower values such as 2C or C/5 to 80, 90 or 100% SOC) would also be possible with the results disclosed herein.

**Experiment 13: Investigation into the electrochemical performance of an alternate polyalkyl phosphate-containing electrolyte sample TEL 40 (not according to the present invention).**

Figure 15 presents the long-term cycling of 2E 10 mAh cells using the TEP-containing TEL 40 electrolyte (not according to the present invention). It can be appreciated that TEP is in the same class of polyalkyl phosphate family as that containing TMP - an embodiment of the present invention discussed in Experiments 7 - 9. From Figure 15, a cycle life of 196 cycles could be obtained to 20 % capacity fade at the C/A mass balance of around 1.4 for cell APFC221 if TEL 40 is used as the electrolyte. Referring to Figure 10 of Experiment 9 as described previously, it can be seen that the cell FPC180622 using TEL 2 with a similar C/A mass balance as used for APFC221 (1.52 vs 1.4) could yield a cycle life of around 175 cycles (TEL 2 is the TMP-based electrolyte). As had been demonstrated in Experiment 9, increasing the C/A mass balance for TMP-based electrolytes drastically improves cycle lives in otherwise like-for-like cells. Since TEP is in the same family of solvents as TMP, it would hence be obvious to anyone skilled in the art that TEP-based electrolytes can also yield longer cycle lives if the C/A mass balance is appropriately adjusted (increased, for this particular cathode/anode combination).

**Experiment 14: Investigation into the use of the diluting solvent HFE sample TEL 41 (not according to the present invention).**

In order to enhance electrolyte performance by decreasing total electrolyte viscosity, Figure 16 compares the long-term cycling of 2E 10 mAh cells using TEL 0 (APFC76) with APFC222 utilising TEL 0 with 50 wt% of the hydrofluoroether HFE (TEL 41). From the prior arts of highly concentrated lithium-ion electrolytes (where salt concentration is typically > 4M), it has recently been established that HFE is a good diluting solvent which does not change the nature of the salt interaction with the base solvent(s) while it is also low cost and has good electrochemical and thermal stability. Reduced overall viscosity of the electrolyte can hence enhance performance of alkali-ion batteries as anyone skilled in the art would be cognizant of. With regards to the concept of dilution, there has not been any prior art for dilute sodium-ion electrolytes which utilise non-metal (sodium) based anodes. Hence, it would not be obvious to anyone skilled in the art that addition of some quantities of this diluting solvent to established dilute electrolytes (such as those discussed previously in this patent disclosure) would enhance the performance of a resulting sodium-ion battery or detract from its performance (it is not trivial to anticipate how his hydrofluoroether would interact with many known sodium-ion non-sodium metal anodes such as carbon-based anodes or alloy-based anodes, to name a couple). With a view to shed light on this matter, referring to Figure 16, it is obvious that APFC222 containing the diluent-based TEL 41 outperforms APFC76 containing the control TEL 0 electrolyte in that it has higher initial discharge capacity (107.5 mAh/g vs 105 mAh/g) and also longer cycle life to 20% capacity fade (cycle 288 vs cycle 265). Hence, it has been established by this Experiment that adding a diluting hydrofluoroether solvent to commercial-type sodium-ion electrolytes enhances the performance of resulting sodium-ion electrolytes even further.

**Experiment 15a: Investigation into the use of the diluting solvent HFE in PC-based electrolytes according to the present invention: small-scale 10 mAh pouch cells.**

Experiment 15a extends Experiment 14 in the domain of sodium-ion electrolytes containing majority PC as the solvent, in line with embodiments of the present invention. Figure 17 presents the long-term cycling of 2E 10 mAh full sodium-ion cells using either TEL 0 (control), TEL 37a (PC-based electrolyte with 2 wt% PCS and 1 wt% P123 surfactant additives), TEL 43 (TEL 37a with 20 wt% of HFE diluent) and TEL 43c (TEL 37a with 40 wt% HFE diluent and 1 m NaPF$_6$). It had previously already been established in Experiment 5a that a sodium-ion electrolyte based on just PC solvent with PCS and P123 additives outperforms the control TEL 0 and TEL 36 (PC-only electrolyte with no additive) electrolytes (refer to Figure 7a). A relevant point to note about Figure 17 is that these 10 mAh full cells are fabricated using low GSM hard carbon anodes with a hard carbon active material wt of around 52 GSM ($5.2\ mg/cm^2$) in accordance with the following patent disclosure ('LIGHT GSM ANODE' with PCT application number PCT/GB2019/053519). Furthermore, as indicated in the figure, all cells used Asahi ND525 separator which is a standard polyolefin-type separator used in commercial lithium/sodium-ion cells. Referring to Figure 17, it can be seen that out of the 4 electrolytes shown, TEL 0 had the lowest cycle life to 20% capacity fade (271 cycles) while TEL 37a actually had the longest cycle life (389 cycles). The two diluent-based electrolytes had, crucially, longer cycle lives than the TEL 0 electrolyte (cycle life of 304 cycles for TEL 43 and 320 cycles for TEL 43c) indicating that such PC+HFE-based electrolytes can outperform the control TEL 0 electrolytes.

**Experiment 15b: Investigation into the use of the diluting solvent HFE in PC-based electrolytes according to the present invention: large-scale 1 Ah pouch cells.**

Experiment 15b extends Experiment 15a into the domain of large-scale sodium-ion ion pouch cells of the type that would actually be used in commercial applications, such as 1 Ah pouch cells. Figure 18 shows long-term cycling of such 1 Ah cells at a $\pm C/3$ rate at 30 °C rate using commercial Celgard 2500 separators (these are also commercially-used polyolefin separators analogous to Asahi ND525 separators and it should be understood that the results obtained from such separators can be extended to all such commercially-used polyolefin separators). Results from 5 different electrolytes have been shown. The control cell using TEL 0 electrolyte (FPC190601) demonstrated a cycle life of 308 cycles to 20 % capacity fade (in the figure legend, the cycle life to 20% capacity fade has been mentioned within parenthesis). The other four electrolytes are according to different embodiments of the present invention. Specifically, the electrolytes based on mixed-carbonate-ester co-solvents of EC:DEC:PC=1:2:1 wt/wt with just PCS additive (TEL 19 (not according to the present invention); FPC190605) or PCS and P123 additives (TEL 24; FPC190607 (according to the present invention)) both outperformed the control cell by 27 - 119 cycles consistent with other previously mentioned disclosures in this patent. With regards to PC-based electrolytes, it should firstly be noted that cell FPC190609 using TEL 37a (being PC-based with no diluent co-solvent) did not cycle at all even during the formation cycles at C/10 rate (0 mAh/g was obtained even at C/10). This result should be compared with the performance of TEL 37a at the 10 mAh scale in Experiment 5a (cell APFC243) where TEL 37a resulted in a cycle life of 389 cycles at a faster cycling rate of $\pm C/2$ despite using similar GSM hard carbon anode and similar C/A mass balances. This result is unique to PC-only electrolyte as the control TEL 0 electrolyte actually delivered slightly longer cycle life at the 1 Ah scale (308 cycles) vs that seen at the 10 mAh scale shown in Figure 17 (271 cycles).

The reason for the non-cycling at all for the PC-based TEL 37a electrolyte at the 1 Ah scale is the well-known relatively higher viscosity of PC solvent - as demonstrated herein, this higher viscosity can still enable small-scale cells (such as 10 mAh pouch cells or Swagelok or coin-cells predominantly used in prior arts) to function effectively but the high viscosity of PC-only electrolytes imposes insurmountable barriers when used in actual commercial-scale cells. In particular, the high viscosity of PC-only electrolytes leads to very poor electrolyte wettability on polyolefin separators that are used in commercial batteries. The only other way to enable such PC-only electrolytes to function in large-scale cells would be to use glass fibre separators that typically hold a large amount of electrolyte per unit weight - this might overcome the wettability issues but would impose a severe weight (and cost) penalty on the resulting battery due to the usage of much more amount of electrolyte in a similar capacity-rated cell vs the case of a battery that uses polyolefin separators. Therefore, the resulting energy density of the battery would be significantly lower - this is obviously not appealing commercially. It should, hence, be appreciated by anyone skilled in the art that prior arts which showed good performance of PC-only electrolytes (with/without small amounts of additives) in small-scale cells are not relevant commercially at all and that the burden of proof of commercial viability of such PC-only electrolytes should lie on the particular prior art to demonstrate using commercially-relevant materials and methodologies at commercial scales (large-scale cells). Keeping this in mind, Figure 18 presents the long-term cycling of TEL 43 (according to the present invention) which is PC-based electrolyte with 20 wt% HFE diluent and 1wt% surfactant P123. As shown, just 20 wt% HFE diluent and 1wt% P123 can enable PC-based electrolytes to function very effectively - in fact, this cell, FPC190610, delivered the longest cycle life by far to 20% capacity fade (610 cycles), almost doubling the cycle life of the control TEL 0 sample in like-for-like conditions.

**Experiment 16: Investigation into the use of the alternate diluting solvent in PC-based electrolytes according to the present invention.**

Experiment 16 provides another example of a diluent that can be used to enable PC-dominant electrolytes to function in large-scale commercial sodium-ion cells. In this example, cycle lives at a fast $\pm 1$ C were investigated at 30 °C within the 4.2 - 1 V window using either HFE-based TEL 43e (FPC200116) or the D2-based TEL 58a (FPC191023) electrolyte, as shown in Figure 19 (in the legend, the cycle life at 20% capacity fade has been indicated). It can be seen that the cycle lives of both electrolytes at this fast 1C rate are higher than those seen even for small-scale cells using the control TEL 0 electrolyte at 4.2 - 1 V: using these diluent-based PC-based electrolytes, cycle lives of around 467 - 562 cycles are achievable which is almost double with respect to the cycle life of 271 cycles if using the control TEL 0 electrolyte (refer to Figure 17 and to cell APFC89) despite favourable conditions for the latter (small-scale 10 mAh cells and at the slower $\pm$C/2 cycling rate where likelihood of sodium plating would be even lesser).

Based on these results, it should be understood that other types of diluent solvents can also elicit such great performance form PC-dominant sodium-ion electrolytes. Some examples include, but not limited to the following: other hydrofluoroethers, low viscosity ethers or carbonate-ester or polyalkyl solvents with appropriate permittivity, inertness/stability and coordination properties such that they do not significantly influence solvation of the salt with PC solvent while still influencing the physical properties of the resulting electrolyte.

**Experiment 17: Investigation into the use of alternate additives into in PC + diluent-based electrolytes according to the present invention.**

Experiment 17 provides examples on using other types of electrolyte additives in PC+diluent-based electrolytes in realistic large-scale 0.1 Ah pouch cells. Figure 20 presents long-term cycling of 0.1 Ah cells at $\pm$C/2 within the 4.2 - 1 V window at 30 °C using TEL 24b, TEL 58a, TEL 43e and two new types of PC+HFE-based electrolytes: TEL 42d (using 1 wt% PCS, 1 wt% P123 and 1wt% TMSB additives), all according to the present invention and TEL 43g (using just 2.5 wt% EC additive), not according to the present invention. It can be seen that the cycling characteristics over 150 - 180 cycles for all cells are quite similar: all PC+HFE-based electrolytes have delivered marginally better performance than TEL 24b with TEL 42d appearing to be the highest performing electrolyte thus far pointing to the beneficial role of replacing half of the PCS additive of TEL 43e with TMSB additive (to form TEL 42d). In terms of cost, EC as an additive would be the most favourable as EC is an expensive solvent that is routinely used in large quantities in commercial lithium-ion and sodium-ion electrolytes. Obviously, in traditional electrolytes, EC is itself present as a co-solvent at volume or weight percent such as 20 or 50 %. In the PC+diluent-based electrolytes disclosed herein, having the option of using EC as a cheap electrolyte additive with decent electrochemical performance opens up possibilities of reducing the cost of resultant batteries, which would ultimately influence the cost/kWh of such battery systems. This is commercially significant.

**Experiment 18: Investigation into the oxidative stability of PC-based electrolytes according to the present invention.**

The move to PC-dominant electrolytes can have another positive effect apart from enhanced cycling stabilities - that of higher oxidative stability of the electrolyte in general which would prevent less amount of gas generation in such cells. It is well known in the prior art, especially for lithium-ion batteries, that EC has excellent reductive stability (at low voltages) but poor oxidative stability (at high voltages): EC is well known to decompose on the surface of the graphite anode used in lithium-ion batteries to form stable a SEI. While it is known that PC has better oxidative stability in lithium-ion systems, PC-only or PC-dominant electrolytes cannot be used in lithium-ion batteries as PC exfoliates the graphite anode resulting in significantly poor cycling performance of such batteries. On the other hand, as mentioned previously and demonstrated here in numerous examples, PC-only or PC-dominant based electrolytes can actually enable more superior cycling of sodium-ion cells than state-of-the-art EC-based sodium-ion electrolytes.

One of the reasons why this occurs will be explained here in Experiment 17 which quantitatively investigates the oxidative stability of four sodium-ion electrolytes through Linear Sweep Voltammetry (LSV) loops. In these LSV experiments, 3E pouch cells were fabricated with the same cathode and anode footprint as used in 10 mAh pouch cells shown at several places in this disclosure, but with one main difference: both the cathode and anode were just pure Al current collector foils. In the LSV experiment, these cells were filled with the concerned electrolyte and subjected to a slow 2 mV/s scan rate to different upper cut-off potentials, before being cycled down to 2.5 V and then beginning the process again. Hence, such an experiment is a cross between a cyclic voltammetry and LSV experiment. The advantage of such a modified LSV experiment is that one can accurately calculate the charge passed due to electrolyte oxidation on Al current collector at different V windows, such as 3.8 - 4.2 V, 4.2 - 4.4 V, 4.4 - 5 V and 5 - 8 V. Since the electrodes are just pure Al current collector foils with no active material, all charge passed should be correlated to electrolyte oxidation in theory. In such an experiment, the lower the charge consumed, the lesser would the electrolyte be prone to oxidation and hence, the better should the electrolyte's performance be when used in actual sodium-ion cells charged to around those voltages. Even though most cells discussed in this document were cycled to 4 V or 4.2 V, it should be appreciated by anyone skilled in the art that electrolyte oxidation is catalysed by the presence of cathode active material and conductive carbon present in the cathode and that the modified LSV experiment represents an ideal-case scenario which is not attainable in real-world settings. As such, it can be understood by anyone skilled in the art that the charge consumed due to electrolyte oxidation at higher potential bands in this modified LSV experiment (such as 4.4 - 5 V and 5 - 8 V) might be more representative to what the electrolyte actually experiences in terms of oxidation in actual sodium-ion cells discussed in this disclosure (which were generally charged to 4 V or 4.2 V) due to the catalytic effects of various components.

With this stated, Figure 21 and Table 3 presents the results of the modified LSV experiments on four electrolytes: TEL 0 and TEL 36 (the control samples) and TEL 24 and TEL 37e, which are embodiments of the present invention.

**TABLE 3**

| Electrolyte | Q(mC): 3.8 - 4.2 V (% wrt TEL 0) | Q (mC): 4.2 - 4.4 V (% wrt TEL 0) | Q (mC): 4.4 - 5 V (% wrt TEL 0) | Q (mC): 5 - 8 V (% wrt TEL 0) | Total Q (mC): 3.8 - 8 V (% wrt TEL 0) |
|---|---|---|---|---|---|
| EC:DEC:PC = 1:2:1 wt/wt-based Solvent System | | | | | |
| TEL 0 (CONTROL) | 1.06 | 0.43 | 2.79 | 150.78 | 155.05 |
| TEL 24 | 1.04 (**-1.8 %**) | 0.40 (**-6.8 %**) | 1.94 (**-30.4 %**) | 74.85 (**-50.4 %**) | 78.23 (**-49.5%**) |
| PC-based Solvent System | | | | | |
| TEL 36 (CONTROL) | 0.39 (**-63.4 %**) | 0.22 (**-49.2 %**) | 1.07 (**-61.4 %**) | 69.67 (**-53.8%**) | 71.35 (**-54 %**) |
| TEL 37e | 0.55 (**-48 %**) | 0.45 (**+4.2 %**) | 1.99 (**-28.6 %**) | 51.23 (**-66 %**) | 54.21 (**-65 %**) |

In Figure 21, as indicated by the arrows, the lower the current at a particular voltage value and the higher the voltage value before complete electrolyte breakdown (indicated by the exponential increase in current above 7 V), the less susceptible will the electrolyte be to oxidation. Such kinds of experiments are heavily influenced by the scan rate of the experiment - faster scan rates such as 10 mV/s (used quite frequently in the literature) tend to underestimate degree of electrolyte oxidation due to kinetic (polarisation) effects. This is why a slow value of 2 mV/s was chosen in this experiment to avoid such complications. From Figure 21, it is qualitatively apparent that the two electrolytes formulated according to embodiments of the current invention along with the PC-based TEL 36 are superior with respect to the control TEL 0 sample.

Table 3 provides quantitative values to the amount of charge passed, Q (in units of mC), in the process of oxidation of the different electrolytes in the various voltage ranges as shown. From Table 3, it becomes obvious that TEL 24, TEL 37e and TEL 36 resulted in significantly lesser amount of charge consumed due to electrolyte oxidation than the values seen for the control TEL 0 electrolyte. For example, within the 4.4 - 5 V range, TEL 24 consumed 30.4 % lesser charge with respect to the charge consumed for TEL 0 indicating the positive effects of the PCS and P123 additives in shielding the base TEL 0 electrolyte from electrolyte oxidation (TEL 24 is simply TEL 0 with PCS and P123 additives). Shifting to PC-dominant electrolytes, TEL 36, being pure PC-based with no additives, resulted in the lowest amount of charge passed within this voltage range (61.4 % lower than the TEL 0 sample) while TEL 37e, being based on PCS, P123 and EC additives, consumed 28.6% lesser charge than TEL 0. However, PC-only or PC-dominant electrolytes also benefit from additives as disclosed in this invention and this can be appreciated by considering the total amount of charge passed across 3.8 - 8 V range: in this full voltage range, TEL 37e actually displayed the lowest electrolyte oxidation and hence, it becomes obvious that additives such as PCS, P123 and EC help in making the PC-based electrolyte more resistant to oxidation. As also seen from Figure 21, the onset of complete electrolyte breakdown occurred at the highest potentials for the two electrolytes according to the present invention: for the EC:DEC:PC=1:2:1 wt/wt solvent system, compare TEL 24 with TEL 0 (for TEL 24, breakdown occurred around 7.3 V vs Na/Na$^+$ but for TEL 0, it occurred at just around 6.8 V vs Na/Na$^+$) while for the PC-dominant electrolyte system, compare TEL 37e with TEL 36 (for TEL 37e, breakdown occurred at the highest value around 7.6 V *vs* Na/Na$^+$ but for TEL 36, it occurred also at just around 6.8 V *vs* Na/Na$^+$). It can therefore be appreciated by anyone skilled in the art that just using PC only as the solvent is not sufficient to result in enhanced oxidative stability of the resultant electrolyte (and cycling performance of the resultant sodium-ion cell); rather, it is the combination of PC with additives and diluents as detailed in this disclosure that leads to superior oxidative stability and electrochemical cycling performance.

Such oxidatively stable electrolytes would also ensure that resultant sodium-ion cells do not exhibit significant gassing - as is well known to those skilled in the art of high voltage battery systems, the high voltages used for such batteries can lead to significant gas generation due to electrolyte oxidation and that this gassing can then have seriously detrimental knock-on effects such as capacity deterioration, metal plating due to uneven pressure distribution in the sealed cells or in the worst case, serious explosions. Hence, such LSV experiments conclusively demonstrate that the additives mentioned in this disclosure can significantly enhance the oxidative stability of sodium-ion electrolytes, irrespective of the solvent system used.

The results contained in Experiments 15 - 18 also demonstrate, for the first time, that PC-dominant based electrolytes, with the right additives and diluting co-solvents as disclosed herein, can function effectively with cycle lives much higher than current state-of-the-art electrolytes in large-scale commercial sodium-ion cells at low temperatures - these results were previously thought to be not achievable.

**Experiment 19: Investigation into the use of alternate salts in sodium-ion electrolytes according to the present invention.**

Figure 22 presents cycling results on large-scale 0.1 Ah pouch cells cycled at $\pm$1C at 30 °C between 4 - 1 V using sodium-ion electrolytes based on NaTFSI salt. As can be seen, over 240 cycles, both TEL 61 (same as TEL 24b but using NaTFSI salt as opposed to NaPF$_6$ salt) and the PC+HFE-based TEL 61b delivered higher capacities than TEL 24b indicating that NaTFSI can be an attractive salt which results in even more superior performance when combined with the appropriate solvents and additives as disclosed herein.

**Experiment 20: Investigation into the use of dual-salts in sodium-ion electrolyte TEL 44b (not according to the present invention).**

Figure 23 presents cycling results on small-scale 10 mAh pouch cells cycled at $\pm$C/2 at 30 °C between 4.2 - 1 V using either TEL 0 (control sample) or the dual-salt based TEL 44b (0.5 m NaPF$_6$ + 0.045 m NaBF$_4$ in a mixture of carbonate-ester, HFE diluent and tetraglyme based five solvent system). Using dual-salts with three different classes of solvent systems led to slightly higher capacities for the resultant sodium-ion cell (around 116 mAh/g for APFC246) with similar cycling stability over 90 cycles as compared to the TEL 0 cell (around 113 mAh/g for APFC 89).

**Experiment 21: Investigation into the thermal stability (flash point testing) of sodium-ion electrolytes according to the present invention.**

The flammability of commercial lithium-ion batteries has been well documented in recent years - there have been various reports on lithium-ion batteries catching fire and exploding in various applications such as different types of consumer electronics or electric vehicles. As is well known to those skilled in the art, one of the major causes for these fires/explosions in lithium-ion batteries is the high flammability of the liquid electrolytes used in them. Simply put, such commercial lithium-ion batteries use electrolytes with low flash points. The flash point is not an inherent physico-chemical property of a liquid, but varies with the type of measurement technique and instrument used. As is well known in this field and described in various prior arts such as in Hess et al. (Journal of The Electrochemical Society, 162 (2) A3084-A3097 (2015)), standardised closed-cup flash point testing methods such as the Abel method (for liquids demonstrating a flash point between -30 to 70 °C) and Pensky-Martens method (for liquids demonstrating a flash point > 40 °C) generally deliver the most accurate results in terms of flash point testing. Hence, herein, these methods are used as well and the results of the flash point measurements shown in Table 4 below.

Referring to Table 4, the first entry is that of a typical commercial lithium-ion electrolyte, LP 30 (control sample). As can be seen, a flash point of 32 °C was obtained for LP 30 indicating that it is a flammable liquid and consistent with prior reports, such as Hess *et* al. Furthermore, two other control sodium-ion control electrolytes (TEL 0+ and 151) were also found to be flammable. The control TEL 0 electrolyte demonstrated a higher flash point of 38.5 °C indicating lower flammability than amongst other control samples. As TEL 24b is based on the same solvent system as the control TEL 0 just with PCS and P123 additives, it also delivered the same flash point value of 38.5 °C indicating that these PCS and P123 additives do not affect the flash point. TEL 14d, being based on the non-flammable TMP solvent, demonstrated a higher flash point of 44 °C indicating its superior thermal stability. Furthermore, as already demonstrated in Experiment 7, TMP-based electrolytes self-extinguish very rapidly even if it catches fire indicating that such TMP-based electrolytes are essentially non-flammable irrespective of the flash point.

As expected, the PC-dominant (TEL 43c, TEL 43e and TEL 58a) or PC+TMP-based (TEL 49d) electrolytes were found to be extremely thermally stable: in fact, no flash points were measured until 120 °C whereupon the tests were ceased due to significant emission of black smoke. There results indicate that these PC-dominant electrolytes would rather decompose as opposed to catch fire. From a battery safety viewpoint, this is very appealing in case there are any catastrophic events which trigger thermal runaway - an electrolyte simply decomposing as opposed to catching fire would prevent explosions from taking place. As another attractive point, increasing the diluent wt% form 20 wt% (for TEL 43e) to 40 wt% (for TEL 43c) did not affect thermal behaviour of the electrolyte. Higher proportion of the diluent in an electrolyte blend would further decrease its viscosity and enhance electrochemical performance without (as the flash point results show) affecting thermal stability. This is obviously a huge benefit from a commercial viewpoint.

In summary, it has been demonstrated that various embodiments of the present invention lead to significantly more thermally stable electrolytes as well apart from enhanced electrochemical performance.

**TABLE 4**

| # | Sample | Flash Point Testing Results | Method |
|---|--------|------------------------------|--------|
| 1 | LP 30 (CONTROL) | 32.0 °C | IP 170 (Abel closed-cup) |
| 2 | TEL 0 (CONTROL) | 38.5 °C | IP 170 (Abel closed-cup) |
| 3 | TEL 0+ (CONTROL) | 37 °C | IP 170 (Abel closed-cup) |
| 4 | 151 (CONTROL) | 26.5 °C | IP 170 (Abel closed-cup) |
| 3 | TEL 14d | 44 °C | IP 170 (Abel closed-cup) |
| 4 | TEL 24b | 38.5 °C | IP 170 (Abel closed-cup) |
| 5 | TEL 43c | No flash point detected till 120 °C; sample emitted black smoke from 90 °C | IP 34 (Pensky Marten closed-cup) |
| 6 | TEL 43e | No flash point detected till 120 °C; sample emitted black smoke from 90 °C | IP 34 (Pensky Marten closed-cup) |
| 7 | TEL 49d | No flash point detected till 120 °C; sample emitted black smoke from 90 °C | IP 34 (Pensky Marten closed-cup) |
| 8 | TEL 58a | No flash point detected till 120 °C; sample emitted black smoke from 90 °C. | IP 34 (Pensky Marten closed-cup) |

**Claims**

1. A non-aqueous electrolyte composition comprising:

   a) one or more sodium-containing salts selected from the group consisting of sodium tetrafluoroborate ($NaBF_4$) and sodium hexafluorophosphate ($NaPF_6$); and
   b) a solvent system which comprises:

   i) a first solvent component which comprises one or more organo carbonate-based solvents; and
   ii) a second solvent component which comprises one or more surfactants in an amount of >0.5 to ≤10% by weight of the solvent system, wherein the one or more surfactants are selected from the group consisting of:

   • anionic surfactants selected from carboxylates; sulfates; sulfonates selected from docusates and alkyl benzene sulfonates; and phosphate esters;
   • cationic surfactants selected from $RN^+H_3Cl^-$, $RN^+ (CH_3)_3Cl^-$, diotadecyldimethylammonium chloride, cetyl pyridinium chloride, benzalkonium chloride, hexadecyl trimethylammonium chloride (CTAC) and hexadecyl trimethylammonium bromide (CTAB); and
   • non-ionic surfactants selected from polyol esters, polyoxyethylene esters, and poloxamers.

2. A non-aqueous electrolyte composition according to claim 1 wherein the solvent system further comprises one or more additional solvents selected from one or more glymes, one or more glycol ether acetates, one or more ionic liquids, one or more nitrile-containing compounds, one or more diluents, one or more sulfur-containing compounds, and one or more flame retardant compounds.

3. A non-aqueous electrolyte composition according to claim 1 or 2 wherein the organo carbonate-based solvents are straight or cyclic compounds comprising a carbonyl group of the formula: $R_1O(C=O)OR_2$, wherein $R_1$ and $R_2$ are independently chosen from hydrogen and a $C_1$ to $C_{20}$-straight or cyclic, branched or unbranched, substituted or unsubstituted alkyl or alkenyl group.

4. A non-aqueous electrolyte composition according to claim 3 wherein the organo carbonate-based solvents are one or more selected from propylene carbonate, diethyl carbonate, dimethyl carbonate, ethylene carbonate, ethyl methyl carbonate, fluoroethylene carbonate and vinylene carbonate.

5.  A non-aqueous electrolyte composition according to claim 1 wherein the one or more surfactants are selected from poloxamer 123, poloxamer 188, poloxamer 407 and F127.

6.  A non-aqueous electrolyte composition according to claim 2 wherein the one or more sulfur-based compounds are selected from sulfone-containing compounds and sultone-containing compounds.

7.  A non-aqueous electrolyte composition according to claim 6 wherein the one or more sulfur-based compounds are selected from 1,3-propanediolcyclic sulfate, 1,3 propanesultone, 1-propene 1,3-sultone and sulfalone.

8.  A non-aqueous electrolyte composition according to any of claims 1 to 7 wherein the first solvent component comprises ethylene carbonate, diethyl carbonate and propylene carbonate in the weight ratio 1:2:1.

9.  A non-aqueous electrolyte composition according to any of claims 1 to 7 wherein the first solvent component comprises ethylene carbonate and diethyl carbonate in the weight ratio 1:1.

10. A non-aqueous electrolyte composition according to any of claims 1 to 7 wherein the first solvent component consists of propylene carbonate.

11. A sodium-ion cell comprising a negative electrode, a positive electrode and a non-aqueous electrolyte composition according to any of claims 1 to 10.

12. A sodium-ion cell according to claim 11 wherein the negative electrode comprises a non-graphitisable carbon material.

13. A sodium-ion cell according to claim 12 wherein the negative electrode comprises hard carbon.

14. An energy storage device comprising a negative electrode, a positive electrode and a non-aqueous electrolyte composition according to any of claims 1 to 10.

15. An energy storage device according to claim 14 selected from a battery, a rechargeable battery, an electrochemical device and an electrochromic device.

**Patentansprüche**

1.  Nichtwässrige Elektrolytzusammensetzung, umfassend:

    a) ein oder mehrere natriumhaltige Salze, ausgewählt aus der Gruppe, bestehend aus Natriumtetrafluoroborat ($NaBF_4$) und Natriumhexafluorophosphat ($NaPF_6$); und
    b) ein Lösungsmittelsystem, das Folgendes umfasst:

    i) eine erste Lösungsmittelkomponente, die ein oder mehrere Lösungsmittel auf Organocarbonatbasis umfasst; und
    ii) eine zweite Lösungsmittelkomponente, die ein oder mehrere Tenside in einer Menge von >0,5 bis ≤10 Gew.-% des Lösungsmittelsystems umfasst, wobei das eine oder die mehreren Tenside ausgewählt sind aus der Gruppe, bestehend aus:

    • anionische Tenside, ausgewählt aus Carboxylaten; Sulfaten; Sulfonaten, ausgewählt aus Docusaten und Alkylbenzolsulfonaten; und Phosphatestern;
    • kationische Tenside, ausgewählt aus $RN^+H_3Cl^-$, $RN^+(CH_3)_3Cl^-$, Diotadecyldimethylammoniumchlorid, Cetylpyridiniumchlorid, Benzalkoniumchlorid, Hexadecyltrimethylammoniumchlorid (CTAC) und Hexadecyltrimethylammoniumbromid (CTAB); und
    • nichtionische Tenside, ausgewählt aus Polyolestern, Polyoxyethylenestern und Poloxameren.

2.  Nichtwässrige Elektrolytzusammensetzung nach Anspruch 1, wobei das Lösungsmittelsystem ferner ein oder mehrere zusätzliche Lösungsmittel umfasst, die ausgewählt sind aus einem oder mehreren Glymen, einem oder meh-

**EP 3 977 546 B1**

reren Glykoletheracetaten, einer oder mehreren ionischen Flüssigkeiten, einer oder mehreren nitrilhaltigen Verbindungen, einem oder mehreren Verdünnungsmitteln, einer oder mehreren Schwefelhaltigen Verbindungen und einer oder mehreren flammhemmenden Verbindungen.

3. Nichtwässrige Elektrolytzusammensetzung nach Anspruch 1 oder 2, wobei die Lösungsmittel auf Organocarbonatbasis gerade oder zyklische Verbindungen sind, die eine Carbonylgruppe der Formel umfassen: $R_1O(C=O)OR_2$, wobei $R_1$ und $R_2$ unabhängig ausgewählt sind aus Wasserstoff und einer $C_1$ bis $C_{20}$ -geraden oder zyklischen, verzweigten oder unverzweigten, substituierten oder unsubstituierten Alkyl- oder Alkenylgruppe.

4. Nichtwässrige Elektrolytzusammensetzung nach Anspruch 3, wobei die Lösungsmittel auf Organocarbonatbasis eines oder mehrere sind, die aus Propylencarbonat, Diethylcarbonat, Dimethylcarbonat, Ethylencarbonat, Ethylmethylcarbonat, Fluorethylencarbonat und Vinylencarbonat ausgewählt sind.

5. Nichtwässrige Elektrolytzusammensetzung nach Anspruch 1, wobei das eine oder die mehreren Tenside aus Poloxamer 123, Poloxamer 188, Poloxamer 407 und F127 ausgewählt sind.

6. Nichtwässrige Elektrolytzusammensetzung nach Anspruch 2, wobei die eine oder die mehreren schwefelbasierten Verbindungen aus sulfonhaltigen Verbindungen und sultonhaltigen Verbindungen ausgewählt sind.

7. Nichtwässrige Elektrolytzusammensetzung nach Anspruch 6, wobei die eine oder die mehreren schwefelbasierten Verbindungen aus 1,3-Propandiol-cyclischem Sulfat, 1,3-Propansulton, 1-Propen-1,3-sulton und Sulfon ausgewählt sind.

8. Nichtwässrige Elektrolytzusammensetzung nach einem der Ansprüche 1 bis 7, wobei die erste Lösungsmittelkomponente Ethylencarbonat, Diethylcarbonat und Propylencarbonat im Gewichtsverhältnis 1:2:1 umfasst.

9. Nichtwässrige Elektrolytzusammensetzung nach einem der Ansprüche 1 bis 7, wobei die erste Lösungsmittelkomponente Ethylencarbonat und Diethylcarbonat im Gewichtsverhältnis 1:1 umfasst.

10. Nichtwässrige Elektrolytzusammensetzung nach einem der Ansprüche 1 bis 7, wobei die erste Lösungsmittelkomponente aus Propylencarbonat besteht.

11. Natriumionenzelle, umfassend eine negative Elektrode, eine positive Elektrode und eine nichtwässrige Elektrolytzusammensetzung nach einem der Ansprüche 1 bis 10.

12. Natriumionenzelle nach Anspruch 11, wobei die negative Elektrode ein nicht graphitisierbares Kohlenstoffmaterial umfasst.

13. Natriumionenzelle nach Anspruch 12, wobei die negative Elektrode harten Kohlenstoff umfasst.

14. Energiespeichervorrichtung, umfassend eine negative Elektrode, eine positive Elektrode und eine nichtwässrige Elektrolytzusammensetzung nach einem der Ansprüche 1 bis 10.

15. Energiespeichervorrichtung nach Anspruch 14, ausgewählt aus einer Batterie, einer wiederaufladbaren Batterie, einer elektrochemischen Vorrichtung und einer elektrochromen Vorrichtung.

**Revendications**

1. Composition d'électrolyte non-aqueuse, comprenant :

a) un ou plusieurs sels contenant du sodium choisis dans le groupe constitué par le tétrafluoroborate de sodium ($NaBF_4$) et l'hexafluorophosphate de sodium ($NaPF_6$) ; et
b) un système de solvants qui comprend :

i) un premier composant solvant qui comprend un ou plusieurs solvants à base d'organocarbonates ; et
ii) un deuxième composant solvant qui comprend un ou plusieurs tensioactifs en une quantité > 0,5 à ≤ 10 % en poids du système de solvants, dans laquelle l'un ou plusieurs tensioactifs sont choisis dans le groupe

25

constitué par :

- des tensioactifs anioniques choisis parmi les carboxylates ; les sulfates ; les sulfonates choisis parmi les docusates et les sulfonates d'alkylbenzène ; et les esters de phosphate ;
- des tensioactifs cationiques choisis parmi $RN^+H_3Cl^-$, $RN^+(CH_3)_3Cl^-$, le chlorure de diotadécyldiméthylammonium, le chlorure de cétylpyridinium, le chlorure de benzalkonium, le chlorure d'hexadécyltriméthylammonium (CTAC) et le bromure d'hexadécyltriméthylammonium (CTAB) ; et
- des tensioactifs non ioniques choisis parmi les esters de polyol, les esters de polyoxyéthylène et les poloxamères.

2. Composition d'électrolyte non-aqueuse selon la revendication 1, dans laquelle le système de solvants comprend en outre un ou plusieurs solvants supplémentaires choisis parmi un ou plusieurs glymes, un ou plusieurs acétates d'éther de glycol, un ou plusieurs liquides ioniques, un ou plusieurs composés contenant du nitrile, un ou plusieurs diluants, un ou plusieurs composés contenant du soufre et un ou plusieurs composés ignifuges.

3. Composition d'électrolyte non-aqueuse selon la revendication 1 ou 2, dans laquelle les solvants à base d'organocarbonates sont des composés linéaires ou cycliques comprenant un groupe carbonyle de formule : $R_1O(C=O)OR_2$, où $R_1$ et $R_2$ sont indépendamment choisis parmi l'hydrogène et un groupe alkyle ou alcényle en $C_1$ à $C_{20}$ linéaire ou cyclique, ramifié ou non ramifié, substitué ou non substitué.

4. Composition d'électrolyte non-aqueuse selon la revendication 3, dans laquelle les solvants à base d'organocarbonates sont un ou plusieurs choisis parmi le carbonate de propylène, le carbonate de diéthyle, le carbonate de diméthyle, le carbonate d'éthylène, le carbonate d'éthyle-méthyle, le carbonate de fluoroéthylène et le carbonate de vinylène.

5. Composition d'électrolyte non-aqueuse selon la revendication 1, dans laquelle l'un ou plusieurs tensioactifs sont choisis parmi le poloxamère 123, le poloxamère 188, le poloxamère 407 et le F127.

6. Composition d'électrolyte non-aqueuse selon la revendication 2, dans laquelle l'un ou plusieurs composés à base de soufre sont choisis parmi les composés contenant de la sulfone et les composés contenant de la sultone.

7. Composition d'électrolyte non-aqueuse selon la revendication 6, dans laquelle l'un ou plusieurs composés à base de soufre sont choisis parmi le sulfate 1,3-propanediolcyclique, le 1,3-propanesultone, le 1-propène 1,3-sultone et le sulfalone.

8. Composition d'électrolyte non-aqueuse selon l'une quelconque des revendications 1 à 7, dans laquelle le premier composant solvant comprend du carbonate d'éthylène, du carbonate de diéthyle et du carbonate de propylène dans le rapport pondéral 1:2:1.

9. Composition d'électrolyte non-aqueuse selon l'une quelconque des revendications 1 à 7, dans laquelle le premier composant solvant comprend du carbonate d'éthylène et du carbonate de diéthyle dans le rapport pondéral 1:1.

10. Composition d'électrolyte non-aqueuse selon l'une quelconque des revendications 1 à 7, dans laquelle le premier composant solvant est constitué de carbonate de propylène.

11. Cellule de ions sodium comprenant une électrode négative, une électrode positive et une composition d'électrolyte non-aqueuse selon l'une quelconque des revendications 1 à 10.

12. Cellule de ions sodium selon la revendication 11, dans laquelle l'électrode négative comprend une matière carbonée non graphitisable.

13. Cellule de ions sodium selon la revendication 12, dans laquelle l'électrode négative comprend du carbone dur.

14. Dispositif de stockage d'énergie comprenant une électrode négative, une électrode positive et une composition d'électrolyte non-aqueuse selon l'une quelconque des revendications 1 à 10.

15. Dispositif de stockage d'énergie selon la revendication 14, choisi parmi une batterie, une batterie rechargeable, un dispositif électrochimique et un dispositif électrochrome.

**FIGURE 1a**

**FIGURE 1b**

**FIGURE 2a**

**FIGURE 2b**

FIGURE 3

FIGURE 4

FIGURE 5a

FIGURE 5b

**FIGURE 6**

**FIGURE 7a**

FIGURE 7b

FIGURE 8

FIGURE 9

FIGURE 10

**FIGURE 11**

**FIGURE 12**

**FIGURE 13**

**FIGURE 14a**

FIGURE 14b

FIGURE 15

**FIGURE 16**

**FIGURE 17**

FIGURE 18

FIGURE 19

FIGURE 20

FIGURE 21

**FIGURE 22**

**FIGURE 23**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2019053519 W

**Non-patent literature cited in the description**

- **HESS et al.** *Journal of The Electrochemical Society,* 2015, vol. 162 (2), A3084-A3097